# EUROPEAN PATENT APPLICATION

(11) **EP 4 782 537 A1**
(43) Date of publication of application: **29.07.2026**
(21) Application number: 24867563.9
(22) Date of filing: 20.09.2024
(51) Int. Cl.: C12N 5/10, C12N 15/12, C12N 15/62, A61K 35/545, A61P 35/00, A61P 37/06

(54) **LOW-IMMUNOGENICITY CELL EXPRESSING MCL1 AND PREPARATION METHOD THEREFOR**

(30) Priority: 20.09.2023 CN 202311219098
(71) Applicant: Xellsmart Bio-Pharmaceutical (Suzhou) Co., Ltd., Suzhou, Jiangsu 215332 (CN)
(72) Inventor: LI, Xiang, Suzhou, Jiangsu 215332 (CN); ZHU, Minzhe, Suzhou, Jiangsu 215332 (CN)
(74) Representative: Schnappauf, Georg
(86) International application number: PCT/CN2024/120109
(87) International publication number: WO 2025/061158

(57) **Abstract**

A low-immunogenicity cell expressing MCL1, and a preparation method therefor. The low-immunogenicity pluripotent cell can significantly reduce or escape immune system recognition and attack without changing the renewal and differentiation characteristics of stem cells, especially T-cell, natural killer cell, macrophage and ADCC attack.

## Description

The present application claims priority to Chinese Patent Application No. 2023112190985 filed on September 20, 2023, which is incorporated herein by reference in its entirety.

### TECHNICAL FIELD

The present disclosure belongs to the technical field of genetic engineering and stem cells and particularly relates to an MCL1-expressing hypoimmunogenic cell and a preparation method therefor.

### BACKGROUND

Stem cells are a class of cells capable of self-renewal and differentiation into specific functional somatic cells. Based on the varying degrees of stem cell characteristics, stem cells are primarily classified into: totipotent stem cells, pluripotent stem cells, and adult stem cells. Induced pluripotent stem cells (iPSCs) have the potential to proliferate indefinitely, self-renew, and differentiate into various types of cells, showing important application prospects in the treatment of diseases such as cancer, nerve-related diseases, cardiovascular diseases, etc. However, key problems, such as immunological incompatibility, immunological rejection suffered by transplanted cells, etc., preclude the clinical application of allogeneic functional cell transplantation for treatment.

The human major histocompatibility complex (MHC), also known as the human leukocyte antigen (HLA), is the primary cause of immunological incompatibility. The MHC consists of a range of genes that can be classified into class I, class II, and class III. MHC-I genes are expressed in almost all tissue cell types. Transplanted cells expressing "non-self" MHC class I molecules will stimulate the activation of CD8+ T cells, leading to their elimination. CD4+ helper T cells recognize the MHC-II genes of "non-self" cells, thereby triggering immunological rejection, while class III molecules do not participate in immune activities. In recent years, there have been reports that knocking out genes, such as β-2-microglobulin (B2M), class II major histocompatibility complex transactivator (CIITA), etc., can eliminate the expression of MHC-I and MHC-II on the cell surface or their genes, thereby enabling the cells to have immune tolerance or escape T cell/B cell-specific immune responses and producing immune-compatible hypoimmunogenic pluripotent stem cells.

Myeloid cell leukemia-1 (Mcl-1) (encoded by the MCL1 gene), a member of the anti-apoptotic family, is expressed in a variety of tissues and tumor cells and affects the development of a variety of malignant tumors. It is involved in the regulation of apoptosis and cell survival, and maintains viability but does not maintain proliferation. Its action is mediated through interaction with many other apoptosis regulatory factors. It has been demonstrated that inhibiting Mcl-1 expression is important for increasing apoptosis and regulating tumor disease.

### SUMMARY

Aiming at the defects in the prior art, the present disclosure provides a new method for engineering cells to obtain low immunogenicity. The method can significantly decrease the recognition and attack of transplanted cells by the immune system or enable transplanted cells to escape recognition and attack by the immune system, especially the attack by natural killer cells, T cells, and macrophages, by overexpressing the MCL1 gene in stem cells. The present disclosure proposes a feasible strategy for enabling the immune privilege of cells by developing new genes through unpredictable domain translation.

In the present disclosure, B2M/CIITA double allele knockout (DKO) positive clone cells are successfully constructed by knocking out B2Mfrom the endoplasmic reticulum of human pluripotent stem cells and knocking out CIITA, a positive regulatory factor of MHC-II gene transcription, and the MCL1 gene is then overexpressed in the DKO cells using a lentiviral vector. The resulting human pluripotent stem cells can escape not only attacks by T cells but also killing by NK cells and macrophages. Moreover, these hypoimmunogenic pluripotent stem cells retain their stemness, differentiation ability, etc., which are critical biological functions of pluripotent stem cells.

In order to achieve the above objectives, the present disclosure adopts the following technical solutions:
In a first aspect, the present disclosure provides a hypoimmunogenic pluripotent stem cell comprising: reduced endogenous major histocompatibility class I antigen (MHC-I) function compared to a parental pluripotent stem cell, reduced endogenous major histocompatibility class II antigen (MHC-II) function compared to the parental pluripotent stem cell, and reduced sensitivity to NK cell, macrophage, and T cell killing compared to the parental pluripotent stem cell, wherein the reduced sensitivity to NK cell, macrophage, and T cell killing results from increased expression of an MCL1 protein.

In some embodiments, the cell further comprises a modification to increase expression of one or more of the following polypeptides: DUX4, CD27, CD35, CD200, PD-L1, CD47, CD24, CD26, CCL21, Mfge8, and SerpinB9.

In some embodiments, the MHC-I function is reduced by reducing activity of an MHC class I protein or an MHC-I transcription factor.

In some embodiments, the MHC class I protein comprises an HLA-A protein, an HLA-B protein, or an HLA-C protein.

In some embodiments, the MHC-I transcription factor comprises a B2M protein, a TAP1 protein, a TAP2 protein, a TAP-related glycoprotein, or an NLRC5 protein.

In some embodiments, the MHC-I function is reduced by reducing activity of a B2M protein.

In some embodiments, the B2M protein is a human B2M protein comprising the amino acid sequence set forth in SEQ ID NO: 1 or an amino acid sequence having 90% identity to the amino acid sequence set forth in SEQ ID NO: 1;
or the B2M protein is a monkey B2M protein comprising the amino acid sequence with accession No. XP_045251305.2 or an amino acid sequence having at least 90% identity to the amino acid sequence with accession No. XP_045251305.2.

In some embodiments, the MHC-II function is reduced by reducing activity of an MHC class II protein or an MHC-II transcription factor.

In some embodiments, the MHC class II protein comprises an HLA-DR protein, an HLA-DQ protein, or an HLA-DP protein.

In some embodiments, the MHC-II transcription factor comprises a CIITA protein, an RFXANK protein, an RFX5 protein, or an RFXAP protein.

In some embodiments, the MHC-II function is reduced by reducing activity of a CIITA protein.

In some embodiment, the CIITA protein is a human CIITA protein comprising the amino acid sequence set forth in SEQ ID NO: 2 or an amino acid sequence having 90% identity to the amino acid sequence set forth in SEQ ID NO: 2;
or the CIITA protein is a monkey CIITA protein comprising the amino acid sequence with accession No. XP_005591301.3 or an amino acid sequence having at least 90% identity to the amino acid sequence with accession No. XP_005591301.3.

In some embodiments, the MCL1 protein is a human MCL1 protein comprising the amino acid sequence set forth in SEQ ID NO: 3 or an amino acid sequence having 90% identity to the amino acid sequence set forth in SEQ ID NO: 3;
or the MCL1 protein is a monkey MCL1 protein comprising the amino acid sequence with accession No. XP_045220294.2 or an amino acid sequence having at least 90% identity to the amino acid sequence with accession No. XP_045220294.2.

In some embodiments, the hypoimmunogenic pluripotent stem cell comprises: one or more alterations that reduce activity of an endogenous B2M protein; one or more alterations that reduce activity of an endogenous CIITA protein; and one or more alterations that result in the increased expression of the MCL1 protein in the hypoimmunogenic pluripotent stem cell.

In some embodiments, the hypoimmunogenic pluripotent stem cell further comprises one or more alterations that increase expression of a polypeptide selected from: DUX4, CD27, CD35, CD200, PD-L1, CD47, CD24, CD26, CCL21, Mfge8, and SerpinB9.

In some embodiments, the hypoimmunogenic pluripotent stem cell comprises: one or more alterations that inactivate two alleles of an endogenous B2M gene; one or more alterations that inactivate two alleles of an endogenous CIITA gene;
and one or more alterations that result in increased expression of an MCL1 gene in the hypoimmunogenic pluripotent stem cell.

In a second aspect, the present disclosure further provides a method for producing the hypoimmunogenic pluripotent stem cell of the present disclosure, and the method comprises: reducing the endogenousMHC-

Ifunction in the pluripotent stem cell; reducing the endogenous MHC-IIfunction in the pluripotent stem cell; and increasing expression of a protein that reduces sensitivity of the pluripotent stem cell to NK cell, macrophage, and T cell killing, wherein the protein is the MCL1 protein.

In some embodiments, the method comprises: reducing activity of a B2M protein in the pluripotent stem cell; reducing activity of a CIITA protein in the pluripotent stem cell; and increasing the expression of the MCL1 protein in the pluripotent stem cell.

In some embodiments, the method comprises: eliminating activity of two alleles of a B2M gene in the pluripotent stem cell; eliminating activity of two alleles of a CIITA gene in the pluripotent stem cell; and increasing expression of an MCL1 gene in the pluripotent stem cell.

In some embodiments, activity of a B2M protein in the pluripotent stem cell is reduced and/or activity of a CIITA protein in the pluripotent stem cell is reduced by a technology selected from the following:
introduction of a gene expression-modifying molecule, CRISPR, TALEN, ZFN, and homologous recombination technology; preferably, the gene expression-modifying molecule comprises an siRNA, an shRNA, a microRNA, an antisense RNA, an antisense oligonucleotide ASO, or an anti-miRNA oligonucleotide AMO.

In some embodiments, the method further comprises: increasing expression of at least one gene selected from DUX4, CD27, CD35, CD200, PD-L1, CD47, CD24, CD26, CCL21, Mfge8, and SerpinB9 in the pluripotent stem cell.

In some embodiments, the activity of the B2M protein in the pluripotent stem cell is reduced by clustered regularly interspaced short palindromic repeats (CRISPR)/Cas9 gene editing technology.

In some embodiments, the activity of the CIITA protein in the pluripotent stem cell is reduced by clustered regularly interspaced short palindromic repeats (CRISPR)/Cas9 gene editing technology.

In some embodiments, the expression of the MCL1 protein is increased by modifying an endogenous locus. In some embodiments, the expression of the MCL1 protein is increased by expression of a transgene.

In some preferred embodiments, the expression of the MCL1 protein is increased by synthesizing a nucleic acid sequence encoding the MCL1 protein, constructing the nucleic acid sequence into a lentiviral vector, and then introducing at least one copy of an MCL1 gene under the control of a promoter into the pluripotent stem cell by the lentiviral vector.

In some embodiments, the nucleic acid sequence encoding the MCL1 protein comprises the nucleic acid sequence set forth in SEQ ID NO: 4 or a nucleic acid sequence having at least 80% identity to the nucleic acid sequence set forth in SEQ ID NO: 4;
or the nucleic acid sequence encoding the MCL1 protein comprises the nucleic acid sequence with accession No. XM_045364359.1 or a nucleic acid sequence having at least 90% identity to the nucleic acid sequence with accession No. XM_045364359.1.

In a third aspect, the present disclosure provides a composition comprising the hypoimmunogenic cell according to the first aspect or a hypoimmunogenic pluripotent stem cell prepared by the method according to the second aspect. Preferably, the composition further comprises a pharmaceutically acceptable carrier.

In a fourth aspect, the present disclosure further provides use of the hypoimmunogenic pluripotent stem cell according to the first aspect of the present disclosure, a hypoimmunogenic pluripotent stem cell prepared by the method according to the second aspect of the present disclosure, or the composition according to the third aspect of the present disclosure in the preparation of a medicament for preventing or treating a disease requiring cell transplantation.

In some preferred embodiments, the disease comprises acute leukemia, chronic leukemia, lymphoma, myelodysplastic syndrome, multiple myeloma, small cell lung cancer, breast cancer, testicular cancer, neuroblastoma, ovarian cancer, melanoma, aplastic anemia, primary immunodeficiency, systemic lupus erythematosus, rheumatoid arthritis, ankylosing spondylitis, type I diabetes, Parkinson's disease, Alzheimer's disease, spinal cord injury, retinal degenerative disease, stroke, Huntington's disease, amyotrophic lateral sclerosis, atherosclerosis, hypertension, rheumatic heart disease, cardiomyopathy, arrhythmia, congenital heart disease, valvular heart disease, carditis, myocardial infarction, heart failure, aortic aneurysm, peripheral artery disease, type II diabetes, scurvy, hypoglycemia, hyperlipidemia, or osteoporosis.

### BRIEF DESCRIPTION OF THE DRAWINGS

FIG. 1 shows a B2M gene knockout strategy for a B2M/CIITA double knockout cell line (DKO) and the results of a B2M gene knockout validation performed by PCR.
FIG. 2 shows a CIITA gene knockout strategy for a B2M/CIITA double knockout cell line (DKO) and the results of a CIITA gene knockout validation performed by PCR.
FIG. 3 shows the results of an assessment of the expression of B2M and CIITA at an RNA level in B2M/CIITA double allele knockout (DKO) clone H1 cells by qPCR.
FIG. 4 shows the result of the determination of the B2M protein level in B2M/CIITA double allele knockout (DKO) clone H1 cells by Western blotting.
FIG. 5 shows FACS assay results of stimulating WT and DKO cells with IFN-γ to detect HLA type I/II molecules on the surface of H1 cells and iPSCs.
FIG. 6 shows the results of a karyotype test of B2M/CIITA double allele knockout (DKO) clone H1 cells.
FIG. 7A shows the results of the determination of the protein levels of the POU5F1 and NANOG pluripotency genes in H1 cells (WT cells) and H1-derived DKO cells (DKO cells) by immunofluorescence.
FIG. 7B shows the results of an assessment of the expression of the POU5F1, NANOG, and SOX2 pluripotency genes at an RNA level in H1 cells (WT cells) and H1-derived DKO cells (DKO cells) by RT-qPCR.
FIG. 7C shows the results of an assessment of the expression of the SSEA-4 and Tra1-81 pluripotency genes on the surface of H1 cells (WT cells) and H1-derived DKO cells (DKO cells) by flow cytometry.
FIG. 7D shows the results of an assessment of the expression of the SSEA-4, TRA-1-60, TRA-1-81, OCT-4, and SOX2 pluripotency genes on the surface of iPSC-WT and iPSC-DKO cells by flow cytometry.
FIG. 7E shows the results of the determination of the protein levels of the TRA-1-60, TRA-1-81, OCT-4, SOX2, and NANOG pluripotency genes in iPSC-WT cells by immunofluorescence.
FIG. 7F shows the results of the determination of the protein levels of the TRA-1-60, TRA-1-81, OCT-4, SOX2, and NANOG pluripotency genes in iPSC-DKO cells by immunofluorescence.
FIG. 8 shows the results of an assessment of the immune escape function of WT and DKO cells, as well as iPSC-WT and iPSC-DKO cells, by RTCA.
FIG. 9 shows a schematic diagram of the structure of a pGC-EF1a lentiviral vector.
FIG. 10 shows the results of a validation of CD47 overexpression in DKO+CD47 cells of H1 cells, wherein A of FIG. 10 shows the results of the determination of the expression level of CD47 in the DKO+CD47 cell line of H1 cells by FACS, and B of FIG. 10 shows the results of the determination of the expression level of CD47 in the DKO+CD47 cell line of H1 cells by qPCR.
FIG. 11 shows the results of an assessment of the immune escape function of WT cells and derived DKO+CD47 cells of H1 cells by RTCA.
FIG. 12 shows the results of the determination of the overexpression levels of mRNA (A of FIG. 12) and protein (B of FIG. 12) in a constructed iPSC-DKO+MCL1 cell line by RT-PCR and FACS.
FIG. 13 shows the results of an assessment of the killing of iPSC-DKO+MCL1 cells by NK cells by RTCA, wherein B of FIG. 13 is a statistical graph of multiple killings (N = 6).
FIG. 14 shows the results of an assessment of the killing of iPSC-DKO+MCL1 cells by NK cells via the ADCC route by RTCA, wherein B of FIG. 14 is a statistical graph of multiple killings (N = 4).
FIG. 15 shows the results of an assessment of the killing of iPSC-DKO+MCL1 cells by PBMCs by RTCA, wherein B of FIG. 15 is a statistical graph of multiple killings (N = 4). C of FIG. 15 shows photos of iPSC-DKO+MCL1 cells resisting PBMC killing.
FIG. 16 shows the results of an assessment of the killing of iPSC-DKO+MCL1 cells by macrophages by RTCA, wherein B of FIG. 16 is a statistical graph of multiple killings (N = 9 in the iPSC-WT and iPSC-DKO groups, and N = 8 in the iPSC-DKO+MCL1 group).
FIG. 17 shows the results of an assessment of the stemness of iPSC-DKO+MCL1 cells. A of FIG. 17 shows the results of the determination of the protein levels of the OCT4, NANOG, SOX2, TRA-1-60, and TRA-1-81 pluripotency genes in constructed iPSC-DKO+MCL1 cells by immunofluorescence, and B of FIG. 17 shows the results of the determination of the expression levels of the SSEA-4, TRA-1-60, TRA-1-81, and OCT4 pluripotency genes on the surface of constructed iPSC-DKO+MCL1 cells by flow cytometry.
FIG. 18 shows the results of an assessment of the ability of constructed iPSC-DKO+MCL1 cells to differentiate into three germ layers by immunofluorescence.
FIG. 19 shows the results of an assessment of the ability of iPSC-DKO+MCL1 cells to form a teratoma.
FIG. 20 shows the detection of pluripotency genes in NHP-iPSCs by immunofluorescence (part A), and the formation of teratomas with three germ layers (endoderm, mesoderm, and ectoderm) from the cells as demonstrated by immunohistochemistry (part B).
FIG. 21 shows the expression of HLA-I/II in various cells, including NHP iPSC-WT and NHP iPSC-DKO cells, after IFN-gamma stimulation, as measured using a flow cytometer in Example 7.
   **Note: The labels NHP WT, NHP DKO1, NHP DKO2, and NHP DKO+MCL1 in** **FIGs. 22-25** **correspond to NHP iPSC-WT, NHP iPSC-DKO1, NHP iPSC-DKO2, and NHP iPSC-DKO+MCL1, respectively.**
FIG. 22 shows the results of an assessment of the killing of NHP iPSC-WT and iPSC-DKO cells by monkey T cells by RTCA in Example 7.
FIG. 23 shows an assessment of overexpression in NHP iPSC-DKO+MCL1 cells in Example 7.
FIG. 24 shows the results of an assessment of the killing of NHP iPSC-WT and iPSC-DKO cells, as well as an NHP iPSC-DKO+MCL1 cell strain overexpressing the MCL1 protein, by monkey NK cells by RTCA in Example 7.
FIG. 25 shows the results of an assessment of the killing of NHP iPSC-WT and iPSC-DKO cells, as well as an NHP iPSC-DKO+MCL1 cell strain overexpressing the MCL1 protein, by monkey PBMCs by RTCA in Example 7.

### DETAILED DESCRIPTION

### General Definitions and Terms

All the patents, patent applications, scientific publications, manufacturer's instructions and guidelines, and the like cited herein, whether above-mentioned or below-described, are herein incorporated by reference in their entirety. Any content herein should not be construed as an admission that the present disclosure is not entitled to antedate such disclosures.

Unless otherwise specified, the scientific and technical terms used herein have the meanings generally understood by those skilled in the art. Moreover, the terms associated with the protein and nucleic acid chemistry, molecular biology, cell and tissue culture, and microbiology used herein are the terms widely used in the corresponding fields (see, e.g., Molecular Cloning: A Laboratory Manual, 2nd Edition, J. Sambrook et al. eds., Cold Spring Harbor Laboratory Press, Cold Spring Harbor 1989). Moreover, to facilitate a better understanding of the present disclosure, the definitions and explanations of relevant terms are provided below. As used herein, the expressions "include", "comprise", "contain", and "have" are open-ended and mean to include the listed elements, steps, or components but not to exclude other unlisted elements, steps, or components. The expression "consist of..." excludes any element, step, or component that is not designated. The expression "consist essentially of..." means that the scope is limited to the designated elements, steps, or components, in addition to elements, steps, or components that are optionally present and do not significantly affect the essential and novel characteristics of the claimed subject matter. It will be appreciated that the expressions "consist essentially of..." and "consist of..." are encompassed within the meaning of the expression "comprise".

As used herein, unless otherwise specified in the context, the expression in singular form "a", "an", or "the" includes plural referents. The term "one or more" or "at least one" encompasses 1, 2, 3, 4, 5, 6, 7, 8, 9, or more.

The enumeration of numerical ranges herein merely serves as a shorthand representation for individually referencing each different value falling within the range. Unless otherwise specified herein, each individual value is incorporated into this specification as if it were individually enumerated herein.

Unless the contrary is specifically indicated, the numerical values or ranges shown herein are all modified by "about" to represent the enumerated or claimed numerical value or range ± 20%, ± 10%, ± 5%, or ± 3%.

Unless otherwise indicated, in the method steps described herein, references such as 1), 2)..., i), ii)..., or a), b)... are only used as examples of differentiation and do not imply that the method steps are performed in this order.

The term "pluripotent cell" refers to a cell that is capable of self-renewing and proliferating while remaining in an undifferentiated state, and capable of differentiating into a specialized cell by induction in appropriate conditions.

As used herein, the term "pluripotent stem cell" has the potential to differentiate into any one of the following three germ layers: an endoderm (e.g., gastric junction, gastrointestinal tract, lung, etc.), a mesoderm (e.g., muscle, bone, blood, genitourinary tissue, etc.), or an ectoderm (e.g., epidermal tissue and nervous system tissue). As used herein, the term "pluripotent stem cell" also includes "induced pluripotent stem cell" or "iPSC", a pluripotent stem cell derived from a non-pluripotent cell. Exemplary human pluripotent stem cell lines include the H1 human pluripotent stem cell line, the H9 human pluripotent stem cell line, and the iPSC human pluripotent stem cell line. Other exemplary pluripotent stem cell lines include those available from the National Institutes of Health HumanEmbryonic Stem Cell Registry and Howard Hughes Medical Institute HUES collection (as described in Cowan CA, et al. Derivation of embryonic stem-cell lines from human blastocysts. N Engl J Med. 2004 Mar 25;350(13):1353-6).

As used herein, the term "totipotent" refers to the ability of a cell to form a whole organism. For example, in mammals, only the fertilized egg and the blastomere during the first cleavage are totipotent. In one embodiment, the pluripotent stem cell described herein does not have totipotency and cannot form a whole organism.

As used herein, the term "hypoimmunogenic cell" refers to a cell with low immunogenicity that is achieved by engineering an allogeneic cell using gene editing technology to eliminate immunological rejection.

The cell may be derived from, e.g., a human or a non-human mammal. Exemplary non-human mammals include, but are not limited to, mice, rats, cats, dogs, rabbits, guinea pigs, hamsters, sheep, pigs, horses, cows, and non-human primates. In some embodiments, the cell is derived from an adult human or a non-human mammal. In some embodiments, the cell is derived from a neonatal human, an adult human, or a non-human mammal.

As used herein, the term "immunological rejection" or "immunological incompatibility" means that after transplantation to a recipient, allogeneic cells, tissues, or organs are attacked by immune cells of the recipient, such that their normal physiological function cannot be guaranteed. The human major histocompatibility complex (MHC), also known as the human leukocyte antigen (HLA), is the primary cause of "immunological rejection" or "immunological incompatibility".

As used herein, the term "subject" or "patient" refers to any animal; e.g., a domesticated animal, a zoo animal, or a human. A "subject" or "patient" may be a mammal or a bird, such as a dog, a cat, a bird, livestock, or a human. Specific examples of "subject" and "patient" include, but are not limited to, an individual (particularly a human) suffering from a disease or disorder associated with the liver, heart, lung, kidney, pancreas, brain, neural tissue, blood, bone, bone marrow, etc.

The term "hypoimmunogenic pluripotent stem cell" herein refers to a pluripotent stem cell that retains the characteristics of pluripotent stem cells and, when transferred into an allogeneic host, produces reduced immunological rejection. In a preferred embodiment, the hypoimmunogenic pluripotent stem cell does not produce an immune response. Thus, "hypoimmunogenic" refers to an immune response that is significantly reduced or eliminated as compared to the immune response of the parental ("WT") stem cell before immunoengineering. For example, such a hypoimmunogenic cell may be about 2.5%, 5%, 10%, 20%, 30%, 40%, 50%, 60%, 70%, 80%, 90%, 95%, 97.5%, 99%, or more than 99% less susceptible to immunological rejection than a wild-type cell that has not been immunoengineered.

The term "major histocompatibility complex (MHC)" involves a complex of genes that occurs in all vertebrates. MHC proteins or molecules function in the signaling between lymphocytes and antigen-presenting cells in normal immune responses. The human MHC, also known as the HLA, i.e., the human leukocyte antigen, is located on chromosome 6 and includes MHC-I and MHC-II.

The term "MHC-I" or "MHC class I" involves major histocompatibility complex class I proteins or genes. Within the human MHC-I region are the HLA-A, HLA-B, HLA-C, HLA-E, HLA-F, CD1a, CD1b, and CD1c subregions. MHC class I proteins are present on the surface of almost all cells, including most tumor cells. MHC-I proteins are loaded with antigens that are usually derived from endogenous proteins or pathogens present intracellularly, and then presented to cytotoxic T lymphocytes (CTLs, also known as CD8+ T cells). T cell receptors are capable of recognizing and binding to peptides complexed with MHC class I molecules. Each cytotoxic T lymphocyte expresses a unique T cell receptor that is capable of binding to specific MHC/peptide complexes. MHC class I molecules mainly mediate the presentation of endogenous antigens. The term "MHC-II" or "MHC class II" involves major histocompatibility complex class II proteins or genes. MHCII includes 5 proteins, which are HLA-DP, HLA-DM, HLA-DOB, HLA-DQ, and HLA-DR. MHC class II proteins are mainly expressed on antigen-presenting cells such as B cells, mononuclear macrophages, dendritic cells, etc. MHC class II molecules mainly mediate the presentation of exogenous antigens; they present exogenous antigen polypeptide molecules to Th cells (helper T cells), i.e., stimulating CD4+ T cells. The term "MHC/peptide complex" involves a non-covalent complex of a binding domain of an MHC class I or MHC class II molecule and an MHC class I or MHC class II binding peptide.

The term "knockout" herein refers to a process of inactivating a specific gene in a host cell in which it is present, which results in no production of the protein of interest or in an inactive form. As appreciated by those skilled in the art and further described below, this can be achieved in a number of different ways, including removing the nucleic acid sequence from the gene, or interrupting the sequence with other sequences, altering the reading frame, or altering a regulatory component of the nucleic acid. For example, all or part of the coding region of the gene of interest can be removed or replaced with a "nonsense" sequence, all or part of a regulatory sequence (e.g., a promoter) can be removed or replaced, the translational start sequence can be removed or replaced, etc.

As used herein, the terms "decrease" and "reduce" are both generally used to denote a decrease of a statistically significant amount. However, to avoid ambiguity, "decrease" and "reduce" include a decrease of at least 10% compared to a reference level, such as a decrease of at least about 20%, or at least about 30%, or at least about 40%, or at least about 50%, or at least about 60%, or at least about 70%, or at least about 80%, or at least about 90%, or up to 100% (including a 100% decrease) (i.e., a level indicating absence compared to a reference sample), or any decrease between 10% and 100%, compared to a reference level.

The term "knock-in" or "overexpression" herein refers to the process of adding genetic function to a host cell. This results in an increase in the level of the encoded protein. As appreciated by those skilled in the art, this can be achieved in several ways, including adding one or more additional copies of the gene into the host cell or altering a regulatory component of the endogenous gene to increase the expression of the protein. This can be achieved by modifying the promoter, adding a different promoter, adding an enhancer, or modifying other gene expression sequences.

As used herein, the term "increase" is generally used to denote an increase of a statistically significant amount; to avoid any ambiguity, the term "increase" refers to an increase of at least 10% compared to a reference level, such as an increase of at least about 20%, or at least about 30%, or at least about 40%, or at least about 50%, or at least about 60%, or at least about 70%, or at least about 80%, or at least about 90%, or up to 100%

(including a 100% increase), or any increase between 10% and 100%, compared to a reference level; or an increase by a factor of at least about 2, or at least about 3, or at least about 4, or at least about 5, or at least about 10, or any increase by a factor between 2 and 10 or greater than 10, compared to a reference level.

"β-2 microglobulin" or "β2M" or "B2M" protein is a component of MHC class I molecules. The B2M protein is expressed in all nucleated cells (except red blood cells). It can non-covalently bind to the α chain of an MHC-I molecule, attaching to the cell membrane, and can also be released into various tissue fluids.

"CD47 protein" or "integrin-associated protein (IAP)" is an important self-signal that can bind to the N-terminus of the ligand signal regulatory protein α (SIRPα) on immune cells, thereby inhibiting phagocytosis by macrophages and causing immune escape.

"MHC-II transactivator (CIITA) protein" is a key molecule that regulates the expression of MHC-II. The organism regulates the expression level of the MHC II gene mainly by controlling the expression of CIITA. MCL1 is one of the most frequently amplified anti-apoptotic genes in human cancers. It is involved in the regulation of apoptosis and cell survival, and maintains viability but does not maintain proliferation. By interacting with many other apoptosis regulatory factors to mediate its action, the MCL1 gene isoform 1 of the present disclosure can inhibit apoptosis.

As used herein, the term "isogene" refers to the genetic similarity or identity of a host organism and a cellular transplant where there is immunological compatibility; for example, no immune response is produced.

As used herein, the term "allogeneic" refers to the genetic dissimilarity of a host organism and a cellular transplant where an immune response is produced.

As used herein, the term "B2M-/-" means that a diploid cell has inactivated B2M genes in both chromosomes. As used herein, the term "CIITA-/-" means that a diploid cell has inactivated CIITA genes in both chromosomes.

As used herein, the term "polypeptide" refers to a polymer comprising two or more amino acids covalently linked by peptide bonds. A "protein" may comprise one or more polypeptides, wherein the polypeptides interact with one another covalently or non-covalently. Unless otherwise specified, "polypeptide" and "protein" are used interchangeably.

In the context of cells, "wild type" refers to cells found in nature. However, in the context of pluripotent stem cells, as used herein, it also refers to pluripotent stem cells that have not undergone a gene editing procedure to achieve low immunogenicity, e.g., the parental pluripotent stem cells (WT) described herein. In the present disclosure, the parental pluripotent stem cells of the H1 cell line are also referred to as WT, and the parental pluripotent stem cells of the iPSC cell line are also referred to as iPSC-WT.

As used herein, the term "% identity" with respect to sequences refers to the percentage of nucleotides or amino acids that are identical in the optimal alignment between the sequences to be compared. The differences between two sequences can be distributed over a partial region (segment) or the entire length of the sequences to be compared. The identity between two sequences is generally determined after optimally aligning segments or "comparison windows". Optimal alignment can be conducted manually or by means of algorithms known in the art, including but not limited to the local homology algorithms described in Smith and Waterman, 1981, Ads App. Math. 2, 482 and Needleman S B, Wunsch C D., Journal of Molecular Biology, 1970, 48(3):443-453, the search for similarity method described in Pearson and Lipman, 1988, Proc. Natl Acad. Sci. USA 88, 2444, or using computer programs such as GAP, BESTFIT, FASTA, BLAST P, BLAST N, and TFASTA in the Wisconsin Genetics Software Package, Genetics Computer Group, 575 Science Drive, Madison, Wis. For example, the BLASTN or BLASTP algorithm publicly available from the National Center for Biotechnology Information (NCBI) website can be used to determine the percent identity between two sequences.

The % identity is obtained by determining the number of identical positions to which the sequences to be compared correspond, dividing that number by the number of positions compared (e.g., the number of positions in a reference sequence), and multiplying the result by 100. In some embodiments, the degree of identity is given for at least about 50%, at least about 55%, at least about 60%, at least about 65%, at least about 70%, at least about 75%, at least about 80%, at least about 85%, at least about 90%, at least about 95%, or about 100% of a region. In some embodiments, the degree of identity is given for the entire length of the reference sequence. Alignment for determining sequence identity can be performed using tools known in the art, preferably using optimal sequence alignment, e.g., using Align or using standard settings, preferably EMBOSS::needle, Matrix: Blosum62, Gap Open 10.0, or Gap Extend 0.5.

Herein, "nucleotide" includes deoxyribonucleotides and ribonucleotides and derivatives thereof. As used herein, "ribonucleotide" is a constituent substance of ribonucleic acid (RNA) that consists of one molecule of base, one molecule of pentose, and one molecule of phosphoric acid; it refers to a nucleotide having a hydroxyl group at the 2' position of the β-D-ribofuranosyl group. "Deoxyribonucleotide" is a constituent substance of deoxyribonucleic acid (DNA) that also consists of one molecule of base, one molecule of pentose, and one molecule of phosphoric acid; it refers to a nucleotide in which the hydroxyl group at the 2' position of the β-D-ribofuranosyl group is substituted with hydrogen, and it is a main chemical component of a chromosome. "Nucleotide" is generally referred to by a single letter representing its base: "A (a)" refers to adenine-containing deoxyadenylic acid or adenylic acid, "C (c)" refers to cytosine-containing deoxycytidylic acid or cytidylic acid, "G (g)" refers to guanine-containing deoxyguanylic acid or guanylic acid, "U (u)" refers to uracil-containing uridylic acid, and "T (t)" refers to thymine-containing deoxythymidylic acid.

As used herein, the terms "polynucleotide" and "nucleic acid" are used interchangeably to refer to a polymer of deoxyribonucleotides (deoxyribonucleic acid, DNA) or a polymer of ribonucleotides (ribonucleic acid, RNA). "Polynucleotide sequence", "nucleic acid sequence", and "nucleotide sequence" are used interchangeably to represent the order in which the nucleotides in a polynucleotide are arranged. It will be appreciated by those skilled in the art that the coding strand (sense strand) of a DNA and the RNA encoded by it can be considered to have the same nucleotide sequence, and the deoxythymidylic acid in the sequence of the coding strand of the DNA corresponds to the uridylic acid in the sequence of the RNA encoded by it. As used herein, the term "expression" includes the transcription and/or translation of a nucleotide sequence. Thus, expression may involve the production of a transcript and/or a polypeptide. The term "transcription" involves the process of transcribing the genetic code in a DNA sequence into RNA (transcript). The term "*in vitro* transcription" refers to the synthesis of RNA, particularly mRNA, *in vitro* in a cell-free system (e.g., in an appropriate cell extract) (see, e.g., Pardi N., Muramatsu H., Weissman D., Karikó K. (2013). In:Rabinovich P. (eds) Synthetic Messenger RNA and Cell Metabolism Modulation. Methods in Molecular Biology (Methods and Protocols), vol 969. Humana Press, Totowa, NJ.). Vectors that can be used to produce transcripts are also referred to as "transcription vectors", which comprise regulatory sequences required for transcription. The term "transcription" encompasses "*in vitro* transcription".

As used herein, "encoding" refers to the inherent property of a specific nucleotide sequence in a polynucleotide; for example, a gene, a cDNA, or an mRNA can be used as a template for the synthesis of polymers and macromolecules in other biological processes, as long as it has a definite nucleotide sequence or a definite amino acid sequence. Thus, a gene encoding a protein refers to the production of the protein by transcription and translation of the mRNA of the gene in a cell or other biological systems.

Unless otherwise specified, all methods described herein can be implemented in any suitable order.

### Pluripotent Stem Cell

In one aspect, the present disclosure provides a hypoimmunogenic pluripotent stem cell comprising:
reduced endogenous MHC-I function compared to a parental pluripotent stem cell;
reduced endogenous MHC-II function compared to the parental pluripotent stem cell; and
reduced sensitivity to NK cell, macrophage, and T cell killing compared to the parental pluripotent stem cell. Herein, the parental pluripotent stem cell refers to a parental pluripotent stem cell (also referred to herein as "WT" or "iPSC-WT") that has not undergone a gene editing procedure to achieve low immunogenicity before immunoengineering.

In a particularly preferred embodiment, the reduced sensitivity to NK cell, macrophage, and T cell killing results from increased expression of the MCL1 protein.

As appreciated by those skilled in the art, functional reduction can be achieved in various ways, including removing the nucleic acid sequence from the gene, interrupting the sequence with other sequences, or altering a regulatory component of the nucleic acid. For example, all or part of the coding region of the gene of interest can be removed or replaced with a "nonsense" sequence, a frameshift mutation can be made, all or part of a regulatory sequence such as a promoter can be removed or replaced, and the translational start sequence can be deleted or replaced.

As appreciated by those skilled in the art, the reduction of MHCI (HLA I when the cells are derived from human cells) function in pluripotent stem cells can be measured using technologies known in the art and those described below, e.g., FACS technology using labeled antibodies that bind to the HLA complex, e.g., using commercially available HLA-A, HLA-B, and HLA-C antibodies, which bind to human major histocompatibility HLA class I. The reduction of MHCII (HLA II when the cells are derived from human cells) function in pluripotent stem cells can be measured using technologies known in the art and those described below, e.g., FACS technology using labeled antibodies that bind to the HLA complex, e.g., using commercially available HLA-DQ, HLA-DR, and HLA-DP antibodies, which bind to human major histocompatibility HLA class II.

In some embodiments, the MHC-I function is reduced by reducing activity of an MHC class I protein.

In one embodiment, the MHC class I protein comprises the human leukocyte antigen-A (HLA-A) protein, the human leukocyte antigen-B (HLA-B) protein, or the human leukocyte antigen-C (HLA-C) protein.

In some embodiments, the MHC-I function is reduced by reducing activity of an MHC-I transcription factor.

In some preferred embodiments, the MHC-I transcription factor may be selected from: one or more of β2 microglobulin (B2M), transporter associated with antigen processing 1 (TAP1), transporter associated with antigen processing 2 (TAP2), transporter associated with antigen processing (TAP)-associated glycoprotein (Tapasin), or NOD-like receptor family caspase recruitment domain 5 (NLRC5).

In one embodiment, the MHC-I function is reduced by reducing activity of the HLA-A protein.

In one embodiment, the MHC-I function is reduced by knocking out a gene encoding the HLA-A protein.

In one embodiment, the MHC-I function is reduced by reducing activity of the HLA-B protein.

In one embodiment, the MHC-I function is reduced by knocking out a gene encoding the HLA-B protein.

In one embodiment, the MHC-I function is reduced by reducing activity of the HLA-C protein.

In one embodiment, the MHC-I function is reduced by knocking out a gene encoding the HLA-C protein.

In one embodiment, the MHC-I function is reduced by reducing activity of the TAP1 protein.

In one embodiment, the MHC-I function is reduced by knocking out a gene encoding the TAP1 protein.

In one embodiment, the MHC-I function is reduced by reducing activity of the TAP2 protein.

In one embodiment, the MHC-I function is reduced by knocking out a gene encoding the TAP2 protein.

In one embodiment, the MHC-I function is reduced by reducing activity of the Tapasin protein.

In one embodiment, the MHC-I function is reduced by knocking out a gene encoding the Tapasin protein.

In one embodiment, the MHC-I function is reduced by reducing activity of the NLRC5 protein.

In one embodiment, the MHC-I function is reduced by knocking out a gene encoding the NLRC5 protein.

In one preferred embodiment, the MHC-I function is reduced by reducing activity of the B2M protein.

In one embodiment, the B2M protein is a human B2M protein comprising the amino acid sequence set forth in SEQ ID NO: 1 or an amino acid sequence having at least 90%, 91%, 92%, 93%, 94%, 95%, 96%, 97%, 98%, or 99% identity to the amino acid sequence set forth in SEQ ID NO: 1.

In one embodiment, the MHC-I function is reduced by knocking out a gene encoding the B2M protein.

In some embodiments, the MHC-II function is reduced by reducing activity of an MHC class II protein.

In one embodiment, the MHC class II protein comprises the human leukocyte antigen-DR (HLA-DR) protein, the human leukocyte antigen-DQ (HLA-DQ) protein, or the human leukocyte antigen-DP (HLA-DP) protein. In some embodiments, the MHC-II function is reduced by reducing activity of an MHC-II transcription factor. In some preferred embodiments, the MHC-II transcription factor may be selected from: one or more of MHC-II transactivator (CIITA), regulatory factor X-associated ankyrin (RFXANK), regulatory factor X5 (RFX5), and regulatory factor X-associated protein (RFXAP).

In one embodiment, the MHC-II function is reduced by reducing activity of the HLA-DR protein.

In one embodiment, the MHC-II function is reduced by knocking out a gene encoding the HLA-DR protein.

In one embodiment, the MHC-II function is reduced by reducing activity of the HLA-DQ protein.

In one embodiment, the MHC-II function is reduced by knocking out a gene encoding the HLA-DQ protein.

In one embodiment, the MHC-II function is reduced by reducing activity of the HLA-DP protein.

In one embodiment, the MHC-II function is reduced by knocking out a gene encoding the HLA-DP protein.

In one embodiment, the MHC-II function is reduced by reducing activity of the RFXANK protein.

In one embodiment, the MHC-II function is reduced by knocking out a gene encoding the RFXANK protein.

In one embodiment, the MHC-II function is reduced by reducing activity of the RFX5 protein.

In one embodiment, the MHC-II function is reduced by knocking out a gene encoding the RFX5 protein.

In one embodiment, the MHC-II function is reduced by reducing activity of the RFXAP protein.

In one embodiment, the MHC-II function is reduced by knocking out a gene encoding the RFXAP protein.

In one preferred embodiment, the MHC-II function is reduced by reducing activity of the CIITA protein.

In one embodiment, the CIITA protein is a human CIITA protein comprising the amino acid sequence set forth in SEQ ID NO: 2 or an amino acid sequence having at least 90%, 91%, 92%, 93%, 94%, 95%, 96%, 97%, 98%, or 99% identity to the amino acid sequence set forth in SEQ ID NO: 2.

In one embodiment, the MHC-II function is reduced by knocking out a gene encoding the CIITA protein.

In one preferred embodiment, the gene is knocked out using CRISPR technology. In some instances, CRISPR technology is used to introduce small deletions/insertions into the coding region of the gene such that no functional protein is produced, which is often the result of frameshift mutations that result in the production of stop codons such that truncated, non-functional proteins are produced.

Successful reduction of MHC-I (HLA-I when the cells are derived from human cells) and MHC-II (HLA-II when the cells are derived from human cells) function in pluripotent stem cells can be measured using technologies known in the art, e.g., Western blotting, FACS technology, RT-PCR, and qPCR technology using protein antibodies.

In some embodiments, the reduced sensitivity to NK cell, macrophage, and T cell killing results from increased expression of the MCL1 protein in the pluripotent stem cell. This is accomplished in several ways. As appreciated by those skilled in the art, "knock-in" or transgenic technology can be used. In some instances, the increased expression of MCL1 results from one or more MCL1 transgenes.

Thus, in some embodiments, one or more copies of the MCL1 gene are added to the pluripotent stem cell under the control of an inducible or constitutive promoter. In some embodiments, a lentiviral construct is used as described herein or known in the art. As is known in the art, the MCL1 gene can be integrated into the genome of a host cell under the control of a suitable promoter.

In one embodiment, the increased expression of the MCL1 protein results from an MCL1 transgene.

In one embodiment, the MCL1 protein is a human MCL1 protein comprising the amino acid sequence set forth in SEQ ID NO: 3 or an amino acid sequence having at least 90%, 91%, 92%, 93%, 94%, 95%, 96%, 97%, 98%, or 99% identity to the amino acid sequence set forth in SEQ ID NO: 3.

The presence of sufficient MCL1 protein expression can be assayed using known technologies, e.g., those described in the examples, e.g., using Western blots, ELISA assays, or FACS assays. Generally, "sufficient" in this context means an increase in the expression of the MCL1 protein on the surface of the pluripotent stem cell that silences NK cell and T cell killing.

In yet another aspect, the present disclosure further provides a hypoimmunogenic pluripotent stem cell comprising:
one or more alterations that reduce activity of an endogenous B2M protein;
one or more alterations that reduce activity of an endogenous CIITA protein; and
one or more alterations that result in the increased expression of the MCL1 protein in the hypoimmunogenic pluripotent stem cell.

In one embodiment, the hypoimmunogenic pluripotent stem cell comprises:
one or more alterations that inactivate two alleles of an endogenous B2M gene;
one or more alterations that inactivate two alleles of an endogenous CIITA gene; and
one or more alterations that result in increased expression of an MCL1 gene in the hypoimmunogenic pluripotent stem cell.

In one embodiment, the hypoimmunogenic pluripotent stem cell further comprises: one or more alterations that increase the expression level of a gene selected from DUX4, CD27, CD35, CD200, PD-L1, CD47, CD24, CD26, CCL21, Mfge8, and SerpinB9.

As used herein, the term "alteration" or "genetic alteration" refers to causing a change in a cell, e.g., the pluripotent stem cell described herein, which can be achieved, for example, by modifying the genome or introducing a new gene fragment. Herein, modifying the genome refers to modifying the nucleic acid sequence under intracellular or cell-free conditions to produce engineered pluripotent cells and pluripotent stem cells. Exemplary technologies for "alteration" or "genetic alteration" include, but are not limited to, homologous recombination, knock-in, ZFN (zinc finger nuclease), TALEN (transcription activator-like effector nuclease), CRISPR (clustered regularly interspaced short palindromic repeats)/Cas9, and other site-specific nuclease technologies. These technologies enable double-stranded DNA breaks at desired locus sites. These controlled double-strand breaks promote homologous recombination at the specific locus sites. This process focuses on targeting specific sequences of nucleic acid molecules, such as chromosomes, with endonucleases that recognize and bind to the sequences and induce double-stranded breaks in the nucleic acid molecules. The double-strand breaks are repaired by error-prone non-homologous end-joining (NHEJ) or by homologous recombination (HR). Exemplary technologies for "alteration" or "genetic alteration" also include introduction of gene expression-modifying molecules, including but not limited to siRNA, shRNA, microRNA, antisense RNA, antisense oligonucleotides (ASOs), or anti-miRNA oligonucleotides (AMOs). It will be appreciated by those skilled in the art that many different technologies can be used to engineer the pluripotent cells and pluripotent stem cells of the present disclosure to make them hypoimmunogenic.

Generally, these technologies can be used alone or in combination. For example, CRISPR technology is used to reduce the expression of active B2M and/or CIITA proteins in the engineered cells, and a viral technology (e.g., lentivirus) is used to knock in the MCL1 gene. In addition, it will be appreciated by those skilled in the art that these genes can be manipulated in different orders using different technologies. In some embodiments, the one or more alterations that the hypoimmunogenic pluripotent stem cell of the present disclosure comprises can reduce endogenous major histocompatibility class I antigen (MHC-I) function. In some embodiments, the one or more alterations that the hypoimmunogenic pluripotent stem cell of the present disclosure comprises can reduce endogenous major histocompatibility class II antigen (MHC-II) function. In some embodiments, the one or more alterations that the hypoimmunogenic pluripotent stem cell of the present disclosure comprises can reduce the sensitivity to NK cell, macrophage, and T cell killing.

In some embodiments, the one or more alterations that the hypoimmunogenic pluripotent stem cell of the present disclosure comprises can reduce activity of an endogenous B2M protein. In some embodiments, the one or more alterations that the hypoimmunogenic pluripotent stem cell of the present disclosure comprises can reduce activity of an endogenous CIITA protein. In some embodiments, the one or more alterations that the hypoimmunogenic pluripotent stem cell of the present disclosure comprises can increase the expression of the MCL1 protein.

In some embodiments, the one or more alterations that the hypoimmunogenic pluripotent stem cell of the present disclosure comprises can inactivate two alleles of an endogenous B2M gene. In some embodiments, the one or more alterations that the hypoimmunogenic pluripotent stem cell of the present disclosure comprises can inactivate two alleles of an endogenous CIITA gene. In some embodiments, the one or more alterations that the hypoimmunogenic pluripotent stem cell of the present disclosure comprises can increase expression of the MCL1 gene.

In some embodiments, the one or more alterations that the hypoimmunogenic pluripotent stem cell of the present disclosure comprises can inhibit expression of the endogenous B2M protein. In some embodiments, the one or more alterations that the hypoimmunogenic pluripotent stem cell of the present disclosure comprises can inhibit expression of the endogenous CIITA protein.

In some embodiments, the one or more alterations that the hypoimmunogenic pluripotent stem cell of the present disclosure comprises can interfere with the expression of the endogenous B2M protein. In some embodiments, the one or more alterations that the hypoimmunogenic pluripotent stem cell of the present disclosure comprises can interfere with the expression of the endogenous CIITA protein.

In some embodiments, the one or more alterations that the hypoimmunogenic pluripotent stem cell of the present disclosure comprises can reduce the expression of the endogenous B2M protein. In some embodiments, the one or more alterations that the hypoimmunogenic pluripotent stem cell of the present disclosure comprises can reduce the expression of the endogenous CIITA protein.

In some embodiments, the one or more alterations that the hypoimmunogenic pluripotent stem cell of the present disclosure comprises can knock out the endogenous B2M protein. In some embodiments, the one or more alterations that the hypoimmunogenic pluripotent stem cell of the present disclosure comprises can knock out the endogenous CIITA protein.

In one embodiment, the activity of the endogenous B2M protein is reduced by altering the pluripotent stem cell using clustered regularly interspaced short palindromic repeats/Cas ("CRISPR") technology, which is known in the art.

In one embodiment, the activity of the endogenous CIITA protein is reduced by altering the pluripotent stem cell using clustered regularly interspaced short palindromic repeats/Cas ("CRISPR") technology, which is known in the art.

In one embodiment, the two alleles of the endogenous B2M gene are inactivated by altering the pluripotent stem cell using clustered regularly interspaced short palindromic repeats/Cas ("CRISPR") technology, which is known in the art.

In one embodiment, the two alleles of the endogenous CIITA gene are inactivated by altering the pluripotent stem cell using clustered regularly interspaced short palindromic repeats/Cas ("CRISPR") technology, which is known in the art.

An assay for testing whether a gene has been inactivated is known and described herein. In one embodiment, the assay is a Western blot that uses an antibody against the B2M protein or the CIITA protein to probe a cell lysate. In another embodiment, a reverse transcription polymerase chain reaction (RT-PCR) confirms the presence of an inactivating alteration.

In one embodiment, a viral technology known in the art can be used to cause increased expression of the MCL1 gene in the hypoimmunogenic pluripotent stem cell. The viral technology includes, but is not limited to, the use of retroviral vectors, lentiviral vectors, adenoviral vectors, and Sendai virus vectors. In some embodiments, a nucleic acid sequence encoding the MCL1 protein is introduced into a selected site of the cell; the selected site of the cell is a safe harbor gene site such as AAVS1, CCR5, or the like. As used herein, the term "safe harbor gene site" refers to a site that can be used for safe knock-in of a gene and can ensure normal and stable expression of the introduced gene.

In one preferred embodiment, a lentiviral vector is used to cause increased expression of the MCL1 gene in the hypoimmunogenic pluripotent stem cell.

In one embodiment, the hypoimmunogenic pluripotent stem cell is a human pluripotent stem cell.

In one embodiment, the B2M protein is a human B2M protein comprising the amino acid sequence set forth in SEQ ID NO: 1 or an amino acid sequence having at least 90%, 91%, 92%, 93%, 94%, 95%, 96%, 97%, 98%, or 99% identity to the amino acid sequence set forth in SEQ ID NO: 1.

In one embodiment, the CIITA protein is a human CIITA protein comprising the amino acid sequence set forth in SEQ ID NO: 2 or an amino acid sequence having at least 90%, 91%, 92%, 93%, 94%, 95%, 96%, 97%, 98%, or 99% identity to the amino acid sequence set forth in SEQ ID NO: 2.

In one embodiment, the MCL1 protein is a human MCL1 protein comprising the amino acid sequence set forth in SEQ ID NO: 3 or an amino acid sequence having at least 90%, 91%, 92%, 93%, 94%, 95%, 96%, 97%, 98%, or 99% identity to the amino acid sequence set forth in SEQ ID NO: 3.

In one embodiment, the hypoimmunogenic stem cell comprises:
reduced endogenous major histocompatibility class I antigen (MHC-I) function compared to a parental pluripotent stem cell;
reduced endogenous major histocompatibility class II antigen (MHC-II) function compared to the parental pluripotent stem cell; and
reduced sensitivity to NK cell, macrophage, and T cell killing compared to the parental pluripotent stem cell. In one embodiment, a T cell response induced by the hypoimmunogenic pluripotent stem cell is lower than a T cell response induced by the parental pluripotent stem cell, and the parental pluripotent stem cell does not comprise the alterations that reduce the activity of the B2M protein and the CIITA protein and the alterations that cause increased expression of the MCL1 protein. In one embodiment, the T cell responses are measured by determining the killing of the hypoimmunogenic pluripotent stem cell or the parental pluripotent stem cell by T cells using real-time label-free dynamic cell analysis (RTCA).

In one embodiment, a natural killer (NK) cell response induced by the hypoimmunogenic pluripotent stem cell is lower than an NK cell response induced by a B2M/CIITA double allele knockout (DKO) clone cell, and the DKO cell comprises the alterations that reduce the activity of the B2M protein and the CIITA protein but does not comprise the alterations that cause increased expression of the MCL1 protein. In one embodiment, the NK cell responses are measured by determining the IFN-γ level of NK cells incubated with the hypoimmunogenic pluripotent stem cell or the DKO cell *in vitro*. In one embodiment, the NK cell responses are measured by determining the killing of the hypoimmunogenic pluripotent stem cell or the DKO cell by NK cells using real-time label-free dynamic cell analysis (RTCA).

### Method for Producing Hypoimmunogenic Pluripotent Stem Cell of Present Disclosure

The present disclosure further provides a method for producing the hypoimmunogenic pluripotent stem cell of the present disclosure, and the method comprises: reducing endogenous major histocompatibility class I antigen (MHC-I) function in the pluripotent stem cell; reducing endogenous major histocompatibility class II antigen (MHC-II) function in the pluripotent stem cell; and increasing expression of a protein that reduces sensitivity of the pluripotent stem cell to NK cell, macrophage, and T cell killing, wherein the protein is the MCL1 protein.

In some embodiments, the method comprises: reducing activity of a B2M protein in the pluripotent stem cell; reducing activity of a CIITA protein in the pluripotent stem cell; and increasing the expression of the MCL1 protein in the pluripotent stem cell.

In one embodiment, the method comprises: eliminating activity of two alleles of a B2M gene in the pluripotent stem cell; eliminating activity of two alleles of a CIITA gene in the pluripotent stem cell; and increasing expression of an MCL1 gene in the pluripotent stem cell.

In some embodiments, the activity of the B2M protein in the pluripotent stem cell may be reduced by a technology for "alteration" or "genetic alteration" as described above. In some embodiments, the activity of the CIITA protein in the pluripotent stem cell may be reduced by a technology for "alteration" or "genetic alteration" as described above. The technology is, for example, introduction of a gene expression-modifying molecule, clustered regularly interspaced short palindromic repeats (CRISPR) technology, transcription activator-like effector nuclease (TALEN) technology, zinc finger nuclease (ZFN) technology, or homologous recombination technology. In one preferred embodiment, the gene expression-modifying molecule comprises an siRNA, an shRNA, a microRNA, an antisense RNA, an antisense oligonucleotide (ASO), or an anti-miRNA oligonucleotide (AMO).

In some embodiments, a CRISPR/Cas system comprises a Cas protein or a nucleic acid sequence encoding the Cas protein and at least one or two ribonucleic acids (e.g., gRNAs), wherein the ribonucleic acids are capable of directing the Cas protein to a target motif of a target polynucleotide sequence so that the protein hybridizes with the target motif. In some embodiments, a CRISPR/Cas system comprises a Cas protein or a nucleic acid sequence encoding the Cas protein and a single ribonucleic acid or at least one ribonucleic acid (e.g., gRNA) pair, wherein the ribonucleic acid is capable of directing the Cas protein to a target motif of a target polynucleotide sequence so that the protein hybridizes with the target motif.

In some embodiments, the Cas protein comprises one or more amino acid substitutions or modifications. In some embodiments, the one or more amino acid substitutions comprise a conservative amino acid substitution. In some instances, the substitutions and/or modifications may prevent or reduce proteolytic degradation and/or extend the half-life of the polypeptide in a cell. In some embodiments, the Cas protein may comprise a peptide bond replacement (e.g., urea, thiourea, carbamate, or sulfonylurea). In some embodiments, the Cas protein may comprise a naturally occurring amino acid. In some embodiments, the Cas protein may comprise an optional amino acid (e.g., a D-amino acid, a β-amino acid, homocysteine, or phosphoserine). In some embodiments, the Cas protein may comprise a modification to include a moiety (e.g., PEGylation, glycosylation, lipidation, acetylation, or end-capping).

In some embodiments, the Cas protein comprises a core Cas protein. Exemplary Cas core proteins include, but are not limited to, Cas1, Cas2, Cas3, Cas4, Cas5, Cas6, Cas7, Cas8, and Cas9. In some embodiments, the Cas protein comprises a Cas protein of an E. coli subtype (also known as CASS2). Exemplary Cas proteins of the E. coli subtype include, but are not limited to, Cse1, Cse2, Cse3, Cse4, and Cas5e. In some embodiments, the Cas protein comprises a Cas protein of the Ypest subtype (also known as CASS3). Exemplary Cas proteins of the Ypest subtype include, but are not limited to, Csy1, Csy2, Csy3, and Csy4. In some embodiments, the Cas protein comprises a Cas protein of the Nmeni subtype (also known as CASS4). Exemplary Cas proteins of the Nmeni subtype include, but are not limited to, Csn1 and Csn2. In some embodiments, the Cas protein comprises a Cas protein of the Dvulg subtype (also known as CASS1). Exemplary Cas proteins of the Dvulg subtype include, but are not limited to, Csd1, Csd2, and Cas5d. In some embodiments, the Cas protein comprises a Cas protein of the Tneap subtype (also known as CASS7). Exemplary Cas proteins of the Tneap subtype include, but are not limited to, Cst1, Cst2, and Cas5t. In some embodiments, the Cas protein comprises a Cas protein of the Hmari subtype. Exemplary Cas proteins of the Hmari subtype include, but are not limited to, Csh1, Csh2, and Cas5h. In some embodiments, the Cas protein comprises a Cas protein of the Apern subtype (also known as CASS5). Exemplary Cas proteins of the Apern subtype include, but are not limited to, Csa1, Csa2, Csa3, Csa4, Csa5, and Cas5a. In some embodiments, the Cas protein comprises a Cas protein of the Mtube subtype (also known as CASS6). Exemplary Cas proteins of the Mtube subtype include, but are not limited to, Csm1, Csm2, Csm3, Csm435, and Csm5. In some embodiments, the Cas protein comprises a RAMP-type Cas protein. Exemplary RAMP-type Cas16 proteins include, but are not limited to, Cmr1, Cmr2, Cmr3, Cmr4, Cmr5, and Cmr6.

In some embodiments, the Cas protein is a Streptococcus pyogenes Cas9 protein or a functional portion thereof. In some embodiments, the Cas protein is a Staphylococcus aureus Cas9 protein or a functional portion thereof. In some embodiments, the Cas protein is a Streptococcus thermophilus Cas9 protein or a functional portion thereof. In some embodiments, the Cas protein is a Neisseria meningitides Cas9 protein or a functional portion thereof. In some embodiments, the Cas protein is a Treponema denticola Cas9 protein or a functional portion thereof. In some embodiments, the Cas protein is a Cas9 protein from any bacterial species or a functional portion thereof. The Cas9 proteins are members of a type II CRISPR system. The type II CRISPR system generally includes a trans-encoded small RNA (tracrRNA), endogenous ribonuclease 3 (rnc), and Cas proteins. The Cas9 proteins (also known as CRISPR-associated endonuclease Cas9/Csn1) are polypeptides comprising 1388 amino acids.

In one embodiment, the activity of the B2M protein in the pluripotent stem cell is reduced by clustered regularly interspaced short palindromic repeats (CRISPR)/Cas9 gene editing technology.

In one embodiment, the activity of the two alleles of the B2M gene in the pluripotent stem cell is eliminated by clustered regularly interspaced short palindromic repeats (CRISPR)/Cas9 gene editing technology.

In one embodiment, the activity of the CIITA protein in the pluripotent stem cell is reduced by clustered regularly interspaced short palindromic repeats (CRISPR)/Cas9 gene editing technology.

In one embodiment, the activity of the two alleles of the CIITA gene in the pluripotent stem cell is eliminated by clustered regularly interspaced short palindromic repeats (CRISPR)/Cas9 gene editing technology.

In one embodiment, the expression of the MCL1 protein is increased by modifying an endogenous locus. In some embodiments, the endogenous locus is modified by a technology for "alteration" or "genetic alteration" as described above. The technology is, for example, gene knock-in, clustered regularly interspaced short palindromic repeats (CRISPR) technology, transcription activator-like effector nuclease (TALEN) technology, zinc finger nuclease (ZFN) technology, or homologous recombination technology.

In one embodiment, the expression of the MCL1 protein is increased by expression of a transgene. The expression of the MCL1 protein can be increased using transgene expression technologies known in the art, including but not limited to viral technologies, Piggybac transposon technology, and SleepingBeauty transposon technology.

Herein, an expression construct as described herein can be produced using a well-known recombinant technology. In certain embodiments, the nucleic acid sequence encoding the protein of interest may be operably linked to one or more regulatory nucleotide sequences in an expression construct. Regulatory nucleotide sequences are generally appropriate for the host cell and the subject to be treated. Numerous types of suitable expression vectors and suitable regulatory sequences are known in the art for a variety of host cells. Generally, the one or more regulatory nucleotide sequences may include, but are not limited to, promoter sequences, leader sequences or signal sequences, ribosome-binding sites, transcriptional start and termination sequences, translational start and termination sequences, and enhancer or activator sequences. For the expression construct used herein, a constitutive or inducible promoter known in the art may be used. The promoter may be a naturally occurring promoter, or a hybrid promoter that combines elements of more than one promoter. The expression construct may be present in a cell on an episome (e.g., a plasmid), or the expression construct may be inserted into a chromosome. In one specific embodiment, the expression vector includes a selectable marker gene to allow the selection of a transformed host cell. Certain embodiments include an expression vector comprising a nucleotide sequence encoding the protein of interest operably linked to at least one regulatory sequence. Regulatory sequences for use herein include promoters, enhancers, and other expression control elements. In certain embodiments, an expression vector is designed for the selection of the host cell to be transformed, the protein of interest desired to be expressed, the vector's copy number, the ability to control the copy number, or the expression of any other protein encoded by the vector, such as an antibiotic marker. In some embodiments, the promoter is an EF1a promoter.

A viral technology can be used to cause increased expression of the MCL1 gene in the hypoimmunogenic pluripotent stem cell. The viral technology includes, but is not limited to, the use of retroviral vectors, lentiviral vectors, adenoviral vectors, and Sendai virus vectors.

In one preferred embodiment, the expression of the MCL1 protein is increased by synthesizing a nucleic acid sequence encoding the MCL1 protein, constructing the nucleic acid sequence into a lentiviral vector, and then introducing at least one copy of the MCL1 gene under the control of a promoter into the pluripotent stem cell by the lentiviral vector.

In one embodiment, the nucleic acid sequence encoding the MCL1 protein is introduced into a selected site of the genome of the pluripotent stem cell. In one preferred embodiment, the selected site is a safe harbor gene site of AAVS1 or CCR5. As used herein, the term "safe harbor gene site" refers to a site that can be used for safe knock-in of a gene and can ensure normal and stable expression of the introduced gene.

In one embodiment, the MCL1 protein is a human MCL1 protein.

In one embodiment, the nucleic acid sequence encoding the MCL1 protein comprises a nucleic acid sequence set forth in SEQ ID NO: 4 or a nucleic acid sequence having at least 70%, 80%, 85%, 90%, 95%, 96%, 97%, 98%, or 99% identity to the nucleic acid sequence set forth in SEQ ID NO: 4.

### Prevention or Treatment

The present disclosure further provides use of the hypoimmunogenic pluripotent stem cell of the present disclosure or a hypoimmunogenic pluripotent stem cell prepared by the method of the present disclosure in the preparation of a medicament for preventing or treating a disease requiring cell transplantation.

The hypoimmunogenic pluripotent stem cell of the present disclosure or the hypoimmunogenic pluripotent stem cell prepared by the method of the present disclosure can be induced to differentiate into different cells, and can be used for different prophylactic or therapeutic purposes to prevent or treat different diseases. As will be appreciated by those skilled in the art, the differentiation method depends on the desired cell type using known technologies. For example, the cells may be differentiated in suspension and then formulated in a gel matrix form, such as a Matrigel, gelatin, or fibrin/thrombin form, to promote cell viability. Generally, differentiation can be determined by assessing the presence of cell-specific markers, as is known in the art. For example, under certain differentiation conditions, a cell may be differentiated into a cardiomyocyte, a nerve cell, a glial cell, an endothelial cell, a T cell, an NK cell, an NKT cell, a macrophage, a hematopoietic progenitor cell, a mesenchymal cell, an islet cell, a chondrocyte, a retinal pigment epithelial cell, a renal cell, a hepatocyte, a thyroid cell, a skin cell, a blood cell, or an epithelial cell.

In some embodiments, the disease is cancer, including a solid tumor and a hematological tumor. In some embodiments, the solid tumor comprises small cell lung cancer, breast cancer, testicular cancer, neuroblastoma, ovarian cancer, or melanoma. In some embodiments, the hematological tumor comprises acute leukemia, chronic leukemia, lymphoma, myelodysplastic syndrome, or multiple myeloma.

In one embodiment, the disease comprises aplastic anemia.

In one embodiment, the disease comprises a primary immunodeficiency disease.

In some embodiments, the disease is an autoimmune disease comprising systemic lupus erythematosus, rheumatoid arthritis, ankylosing spondylitis, or type I diabetes.

In some embodiments, the disease is a neurodegenerative disease comprising Parkinson's disease, Alzheimer's disease, spinal cord injury, retinal degenerative disease, stroke, Huntington's disease, or amyotrophic lateral sclerosis.

In some embodiments, the disease is a cardiovascular disease comprising atherosclerosis, hypertension, rheumatic heart disease, cardiomyopathy, arrhythmia, congenital heart disease, valvular heart disease, carditis, myocardial infarction, heart failure, aortic aneurysm, or peripheral artery disease.

In some embodiments, the disease is a metabolic disease comprising type II diabetes, scurvy, hypoglycemia, hyperlipidemia, or osteoporosis.

### Differentiation of Pluripotent Stem Cell

The present disclosure provides a pluripotent stem cell that can be differentiated into different cell types for subsequent transplantation into a recipient subject. As will be appreciated by those skilled in the art, differentiated hypoimmunogenic pluripotent cell derivatives can be transplanted using technologies known in the art, depending on the cell type and the ultimate use of these cells.

### 1. Cardiomyocytes differentiated from pluripotent stem cells

The present disclosure provides pluripotent stem cells that can be differentiated into different cardiac cell types for subsequent transplantation or implantation into a subject (e.g., a recipient).

As will be appreciated by those skilled in the art, the differentiation method depends on the desired cell type using known technologies. Exemplary cardiac cell types include, but are not limited to, cardiomyocytes, nodal cardiomyocytes, conductive cardiomyocytes, working cardiomyocytes, cardiomyocyte precursor cells, cardiac stem cells, atrial cardiac stem cells, ventricular cardiac stem cells, epicardial cells, hematopoietic cells, vascular endothelial cells, endocardial endothelial cells, heart valve interstitial cells, and cardiac pacemaker cells.

In some embodiments, cardiomyocyte precursors include cells capable of (without dedifferentiation or reprogramming) producing progeny including mature (end-stage) cardiomyocytes. Generally, cardiomyocyte precursor cells can be identified using one or more markers selected from the GATA-4, Nkx2.5, and MEF-2 transcription factor families.

In some embodiments, the cardiomyocytes are hypoimmunogenic cardiomyocytes.

In some embodiments, the cardiomyocytes described herein are administered to a recipient subject to treat a cardiac disorder selected from: pediatric cardiomyopathy, age-related cardiomyopathy, dilated cardiomyopathy, hypertrophic cardiomyopathy, restrictive cardiomyopathy, chronic ischemic cardiomyopathy, peripartum cardiomyopathy, inflammatory cardiomyopathy, idiopathic cardiomyopathy, other cardiomyopathies, myocardial ischemia-reperfusion injury, ventricular dysfunction, heart failure, congestive heart failure, coronary artery disease, end-stage heart disease, atherosclerosis, ischemia, hypertension, restenosis, angina, rheumatic heart disease, arterial inflammation, cardiovascular disease, myocardial infarction, myocardial ischemia, congestive heart failure, myocardial infarction, myocardial ischemia, cardiac injury, myocardial ischemia, vascular disease, acquired heart disease, congenital heart disease, atherosclerosis, coronary artery disease, conduction system dysfunction, coronary artery dysfunction, pulmonary hypertension, arrhythmia, muscular dysfunction, muscle mass abnormality, muscle degeneration, myocarditis, infective myocarditis, muscle abnormalities induced by drugs or toxins, hypersensitivity myocarditis, and autoimmune endocarditis.

In some embodiments, a method for producing a population of hypoimmunogenic cardiomyocytes from a population of hypoimmunogenic pluripotent (HIP) stem cells by *in vitro* differentiation comprises: (a) culturing the population of HIP cells in a culture medium comprising a GSK inhibitor; (b) culturing the population of HIP cells in a culture medium comprising a WNT antagonist to produce a population of pre-cardiomyocytes; and (c) culturing the population of pre-cardiomyocytes in a culture medium comprising insulin to produce the population of hypoimmunogenic cardiomyocytes. In some embodiments, the GSK inhibitor is CHIR-99021, a derivative thereof, or a variant thereof. In some instances, the GSK inhibitor is at a concentration ranging from about 2 mM to about 10 mM. In some embodiments, the WNT antagonist is IWR1, a derivative thereof, or a variant thereof. In some instances, the WNT antagonist is at a concentration ranging from about 2 mM to about 10 mM.

Other useful methods for differentiating induced pluripotent stem cells or pluripotent stem cells into cardiomyocytes are described in, e.g., US2017/0152485; US2017/0058263; US2017/0002325; US2016/0362661; US2016/0068814; US9,062,289; US7, 897,389; and US7,452,718. Other methods for producing cardiac cells from induced pluripotent stem cells or pluripotent stem cells are described in, e.g., Xu et al., Stem Cells and Development, 2006, 15(5):631-9; Burridge et al., Cell Stem Cell, 2012, 10:16-28; and Chen et al., Stem Cell Res, 2015, 15(2):365-375.

In various embodiments, the hypoimmunogenic cardiomyocytes may be cultured in a culture medium comprising a BMP pathway inhibitor, a WNT signaling activator, a WNT signaling inhibitor, a WNT agonist, a WNT antagonist, an Src inhibitor, an EGFR inhibitor, a PCK activator, a cytokine, a growth factor, a myocardial agent, other compounds, or the like.

The WNT signaling activator includes, but is not limited to, CHIR99021. The PCK activator includes, but is not limited to, PMA. The WNT signaling inhibitor includes, but is not limited to, a compound selected from KY02111, SO3031 (KY01-I), SO2031 (KY02-I), SO3042 (KY03-I), and XAV939. The Src inhibitor includes, but is not limited to, A419259. The EGFR inhibitor includes, but is not limited to, AG1478.

Non-limiting examples of reagents for producing cardiomyocytes from iPSCs include activin A, BMP-4, Wnt3a, VEGF, soluble frizzled proteins, cyclosporin A, angiotensin II, phenylephrine, ascorbic acid, dimethyl sulfoxide, 5-aza-2'-deoxycytidine, etc.

### 2. Nerve cells differentiated from pluripotent stem cells

The present disclosure provides pluripotent stem cells that can be differentiated into different nerve cell types for subsequent transplantation or implantation into a recipient subject. As will be appreciated by those skilled in the art, the differentiation method depends on the desired cell type using known technologies. Exemplary nerve cell types include, but are not limited to, cerebral endothelial cells, neurons, glial cells, etc.

In some embodiments, the nerve cells are administered to a subject to treat Parkinson's disease, Huntington's disease, multiple sclerosis, other neurodegenerative diseases or disorders, attention deficit hyperactivity disorder (ADHD), Tourette syndrome (TS), schizophrenia, psychosis, depression, or other neuropsychiatric disorders. In some embodiments, the nerve cells described herein are administered to a subject to treat or ameliorate stroke.

In some embodiments, neurons and glial cells are administered to a subject with amyotrophic lateral sclerosis (ALS). In some embodiments, cerebral endothelial cells are administered to palliate a symptom or effect of cerebral hemorrhage. In some embodiments, dopaminergic neurons are administered to a patient with Parkinson's disease. In some embodiments, noradrenergic neurons and GABAergic interneurons are administered to a patient who has experienced an epileptic seizure. In some embodiments, motor neurons, interneurons, Schwann cells, oligodendrocytes, and microglial cells are administered to a patient who has experienced spinal cord injury.

In some embodiments, cerebral endothelial cells (ECs), precursors thereof, and progenitor cells thereof are differentiated from pluripotent stem cells (e.g., induced pluripotent stem cells) on a surface by culturing the cells in a culture medium comprising one or more factors that promote the production of cerebral endothelial cells or nerve cells. In some instances, the culture medium comprises one or more of CHIR-99021, VEGF, alkaline FGF, and Y-27632. In some embodiments, the culture medium comprises supplements designed to promote the survival and function of nerve cells. In some examples, cerebral endothelial cells (ECs), precursor cells thereof, and progenitor cells thereof are differentiated from pluripotent stem cells on a surface by culturing the cells in an unconditioned or conditioned culture medium. In some instances, the culture medium comprises a factor or small molecule that promotes differentiation. In some embodiments, the medium comprises one or more factors or small molecules selected from VEGR, FGF, SDF-1, CHIR-99021, Y-27632, SB431542, and any combination thereof. In some embodiments, the surface for differentiation comprises one or more extracellular matrix proteins. In some instances, differentiation is generally determined by assessing the presence of cell-specific markers, as is known in the art. In some embodiments, cerebral endothelial cells express or secrete a factor selected from CD31, VE cadherin, and a combination thereof.

In some embodiments, neurons, precursors thereof, and progenitor cells thereof are differentiated from pluripotent stem cells by culturing the cells in a culture medium comprising one or more factors. The one or more factors are selected from the group consisting of GDNF, BDNF, GM-CSF, B27, alkaline FGF, alkaline EGF, NGF, CNTF, an SMAD inhibitor, a Wnt antagonist, an SHH signaling activator, and any combination thereof. In some embodiments, the SMAD inhibitor is selected from SB431542, LDN-193189, NogginPD169316, SB203580, LY364947, A77-01, A-83-01, BMP4, GW788388, GW6604, SB-505124, lerdebmumab, metebmumab, GC-I008, AP-12009, AP-11014, LY550410, LY580276, LY364947, LY2109761, SB-505124, E-616452 (RepSoxALK inhibitor), SD-208, SMI6, NPC-30345, K26894, SB-203580, SD-093, activin-M108A, P144, soluble TBR2-Fc, DMH-1, dorsomorphin dihydrochloride, and derivatives thereof.

In some embodiments, the Wnt antagonist is selected from the group consisting of XAV939, DKK1, DKK-2, DKK-3, Dkk-4, SFRP-1, SFRP-2, SFRP-5, SFRP-3, SFRP-4, WIF-1, Soggy, IWP-2, IWR1, ICG-001, KY0211, Wnt-059, LGK974, IWP-L6, and derivatives thereof. In some embodiments, the SHH signaling activator is selected from the group consisting of Smoothened agonist (SAG), a SAG analog, SHH, C25-SHH, C24-SHH, purmorphamine, Hg-Ag, and derivatives thereof.

In some embodiments, the stem cells described herein are differentiated into dopaminergic neurons, including dopaminergic progenitor cells. The stem cells are cultured in a differentiation culture medium comprising a supplement or additive to induce neuron differentiation. In some embodiments, the cells are cultured in the presence of a supplement or additive to induce floor plate cells. In some embodiments, the supplement or additive includes the BMP inhibitor LDN193189, the ALK-5 inhibitor A83-01, the Smoothened agonist purmorphamine, FGF8, the GSK3 inhibitor CHIR99021, glial cell line-derived neurotrophic factor, GDNF, ascorbic acid, brain-derived neurotrophic factor (BDNF), dibutyryladenosine monophosphate (dbcAMP), and the ROCK inhibitor Y-27632.

In some embodiments, a method for producing a population of hypoimmunogenic dopaminergic neurons from a population of hypoimmunogenic induced pluripotent stem cells (HIP cells) by *in vitro* differentiation comprises: (a) culturing the population of HIP cells in a first culture medium comprising one or more factors selected from sonic hedgehog (SHH), BDNF, EGF, bFGF, FGF8, WNT1, retinoic acid, a GSK3 inhibitor, and an ALK inhibitor, and a ROCK inhibitor, to produce a population of immature dopaminergic neurons; and (b) culturing the population of immature dopaminergic neurons in a second culture medium different from the first culture medium to produce the population of dopaminergic neurons. In some embodiments, the GSK inhibitor is CHIR-99021, a derivative thereof, or a variant thereof. In some instances, the GSK inhibitor is at a concentration ranging from about 2 mM to about 10 mM. In some embodiments, the ALK inhibitor is SB-431542, a derivative thereof, or a variant thereof. In some instances, the ALK inhibitor is at a concentration ranging from about 1 mM to about 10 mM. In some embodiments, the first culture medium and/or the second culture medium does not comprise animal serum.

Methods for differentiating pluripotent stem cells are described in, e.g., Kikuchi et al., Nature, 2017, 548, 592-596; Kriks et al., Nature, 2011, 547-551; Doi et al., Stem Cell Reports, 2014, 2, 337-50; Perrier et al., Proc Natl Acad Sci USA, 2004, 101, 12543-12548; Chambers et al., NatBiotechnol, 2009, 27, 275-280; and Kirkeby et al., Cell Reports, 2012, 1, 703-714. Useful descriptions of neurons derived from stem cells and preparation methods therefor can be found in, e.g., Kirkeby et al., Cell Rep, 2012, 1:703-714. The contents of Kriks et al., Nature, 2011, 480:547-551; Wang et al., Stem Cell Reports, 2018, 11(1):171-182; Studer L. Strategies for bringing stem cell-derived dopamine neurons to the clinic-The NYSTEM trial. [J]. Progress in Brain Research, 2017, 230:191-212; Liu et al., Nat Protoc, 2013, 8:1670-1679; Upadhya et al., Curr Protoc Stem Cell Biol, 38, 2D.7.1-2D.7.47; and U.S. Patent Application Nos. 20160115448, US8,252,586, US8,273,570, US9,487,752, and US 10,093,897, the contents of which are incorporated herein by reference in their entirety.

In some embodiments, glial cells, including microglia, astrocytes, oligodendrocytes, ependymal cells and Schwann cells, glial precursors, and glial progenitor cells, are produced by differentiating pluripotent stem cells into therapeutically effective glial cells, etc. Differentiation of hypoimmunogenic pluripotent stem cells produces hypoimmunogenic nerve cells, such as hypoimmunogenic glial cells.

In some embodiments, glial cells, precursors, and progenitor cells thereof are produced by culturing pluripotent stem cells in a culture medium comprising one or more reagents selected from retinoic acid, IL-34, M-CSF, FLT3 ligand, GM-CSF, CCL2, a TGFβ inhibitor, a BMP signaling inhibitor, an SHH signaling activator, FGF, platelet-derived growth factor (PDGF), PDGFR-α, HGF, IGF-1, and noggin. In certain instances, the SHH signaling activator is selected from sonic hedgehog (SHH) protein, dorsomorphin, noggin, and any combination thereof. In certain instances, the BMP signaling inhibitor is LDN193189, SB431542, or a combination thereof.

In some embodiments, glial cells are determined to express known glial cell biomarkers. Useful methods for generating glial cells, precursor cells thereof, and progenitor cells thereof from stem cells can be found in, e.g., US7,579,188; US7,595,194; US8,263,402; US8,206,699; US8,252,586; US9,193,951; US9,862,925; US8,227,247; US9,709,553; US2018/0187148; US2017/0198255; US2017/0183627; US2017/0182097; US2017/253856; US2018/0236004; WO2017/172976; and WO2018/093681.

In some embodiments, differentiation of pluripotent stem cells is performed by exposing or contacting cells with a specific factor that is known to produce a specific cell lineage, so as to target their differentiation to a specific, desired lineage and/or cell type.

In some embodiments, terminally differentiated cells exhibit specialized phenotypic properties or characteristics. In certain embodiments, the stem cells described herein are differentiated into a neuroectodermal, neuronal, neuroendocrine, dopaminergic, cholinergic, serotonergic (5-HT), glutamatergic, GABAergic, adrenergic, noradrenergic, sympathetic neuronal, parasympathetic neuronal, sympathetic peripheral neuronal, or glial cell population. In some instances, the glial cell population includes a microglial cell population or a macroglial (central nervous system cell: astrocyte, oligodendrocyte, ependymal cell, and radial glia; and peripheral nervous system cell: Schwann cell and satellite cell) cell population, or precursors and progenitor cells of any of the aforementioned cells.

Protocols for generating different types of nerve cells are described in PCT Application No. WO2010144696 and U.S. Patent Nos. 9,057,053; 9,376,664; and 10,233,422. Additional descriptions of methods for differentiating hypoimmunogenic pluripotent cells can be found in, e.g., Deuse et al., Nature Biotechnology, 2019, 37, 252-258 and Han et al., Proc Natl Acad Sci USA, 2019, 116(21), 10441-10446. Methods for determining the effect of nerve cell transplantation in an animal model of a neurological disease or disorder are described in the following references: for spinal cord injury-Curtis et al., Cell Stem Cell, 2018, 22, 941-950; for Parkinson's disease-Kikuchi et al., Nature, 2017, 548:592-596; for ALS-Izrael et al., Stem Cell Research, 2018, 9(1):152 and Izrael et al., IntechOpen, DOI: 10.5772/intechopen.72862; for epilepsy-Upadhya et al., PNAS, 2019, 116(1):287-296. The efficacy of nerve cell transplantation for spinal cord injury can be assessed in, e.g., a rat model of acute spinal cord injury, as described in McDonald et al., Nat. Med., 1999, 5:1410 and Kim, et al., Nature, 2002, 418:50. For example, successful transplantation may show the presence of transplant-derived cells in the lesion 2-5 weeks later, which are differentiated into astrocytes, oligodendrocytes, and/or neurons and migrate along the spinal cord from the lesioned end, and improvements in gait, coordination, and weight-bearing. Specific animal models are selected based on the nerve cell type and the neurological disease or disorder to be treated. Nerve cells can be administered in a manner that permits them to engraft to the intended tissue site and reconstitute or regenerate the functionally deficient area. For example, according to the disease being treated, the nerve cells can be directly transplanted into a parenchymal or intrathecal site of the central nervous system. In some embodiments, any of the nerve cells described herein, including cerebral endothelial cells, neurons, dopaminergic neurons, ependymal cells, astrocytes, microglial cells, oligodendrocytes, and Schwann cells, are injected into a patient intravenously, intraspinally, intracerebroventricularly, intrathecally, intraarterially, intramuscularly, intraperitoneally, subcutaneously, intramuscularly, intraabdominally, intraocularly, retrobulbarly, and combinations thereof. In some embodiments, the cells are injected or deposited in the form of a bolus injection or continuous infusion. In certain embodiments, the nerve cells are administered by injection into the brain, near the brain, and combinations thereof. For example, the injection can be performed through a burr hole made in the subject's skull. Suitable sites for administration of nerve cells to the brain include, but are not limited to, the cerebral ventricles, lateral ventricles, cisterna magna, putamen, nucleus basalis, hippocampal cortex, striatum, caudate regions of the brain, and combinations thereof.

Additional descriptions of nerve cells, including dopaminergic neurons, for use in the present disclosure can be found in WO2020/018615, the disclosures of which are incorporated herein by reference in their entirety.

### 3. Endothelial Cells Differentiated from Pluripotent Stem Cells

The present disclosure provides pluripotent stem cells that can be differentiated into various endothelial cell types for subsequent transplantation or implantation into a subject (e.g., a recipient). As will be appreciated by those skilled in the art, the differentiation method depends on the desired cell type using known technologies. Exemplary endothelial cell types include, but are not limited to, capillary endothelial cells, vascular endothelial cells, aortic endothelial cells, arterial endothelial cells, venous endothelial cells, renal endothelial cells, cerebral endothelial cells, and hepatic endothelial cells.

In some embodiments, the endothelial cells described herein are administered to a recipient subject to treat a vascular disorder selected from the group consisting of: vascular injury, cardiovascular disease, vascular disease, peripheral vascular disease, ischemic disease, myocardial infarction, congestive heart failure, peripheral vascular occlusive disease, hypertension, ischemic tissue injury, reperfusion injury, limb ischemia, stroke, neuropathy (e.g., peripheral neuropathy or diabetic neuropathy), organ failure (e.g., liver failure or kidney failure), diabetes, rheumatoid arthritis, osteoporosis, cerebrovascular disease, hypertension, angina and myocardial infarction induced by coronary heart disease, renal vascular hypertension, kidney failure induced by renal artery stenosis, lower extremity claudication, and other vascular conditions or diseases.

In some embodiments, the hypoimmunogenic pluripotent cells are differentiated into endothelial colony forming cells (ECFCs) to form new blood vessels to address a peripheral artery disease. Technologies for differentiating endothelial cells are known. See, e.g., Prasain et al., doi: 10.1038/nbt.3048, the contents of which are incorporated herein by reference in their entirety and specifically for methods and reagents for the production of endothelial cells from human pluripotent stem cells, and also for transplantation technologies. Differentiation can be determined as is known in the art, generally by assessing the presence of endothelial cell-associated or specific markers or by functional measurement.

In some embodiments, a method for producing a population of hypoimmunogenic endothelial cells from a population of hypoimmunogenic pluripotent cells by *in vitro* differentiation comprises: (a) culturing the population of HIP cells in a first culture medium comprising a GSK inhibitor; (b) culturing the population of HIP cells in a second culture medium comprising VEGF and bFGF to produce a population of pre-endothelial cells; and (c) culturing the population of pre-endothelial cells in a third culture medium comprising a ROCK inhibitor and an ALK inhibitor to produce the population of hypoimmunogenic endothelial cells.

In some embodiments, the GSK inhibitor is CHIR-99021, a derivative thereof, or a variant thereof. In some instances, the GSK inhibitor is at a concentration ranging from about 1 mM to about 10 mM. In some embodiments, the ROCK inhibitor is Y-27632, a derivative thereof, or a variant thereof. In some instances, the ROCK inhibitor is at a concentration ranging from about 1 pM to about 20 pM. In some embodiments, the ALK inhibitor is SB-431542, a derivative thereof, or a variant thereof. In some instances, the ALK inhibitor is at a concentration ranging from about 0.5 pM to about 10 pM.

In some embodiments, the first culture medium comprises 2 pM to about 10 pM CHIR-99021. In some embodiments, the second culture medium comprises 50 ng/mL VEGF and 10 ng/mL bFGF. In other embodiments, the second culture medium further comprises Y-27632 and SB-431542. In various embodiments, the third culture medium comprises 10 pM Y-27632 and 1 pM SB-431542. In certain embodiments, the third culture medium further comprises VEGF and bFGF. In particular instances, the first culture medium and/or the second culture medium does not comprise insulin.

In some embodiments, the population of hypoimmunogenic endothelial cells is isolated from non-endothelial cells. In some embodiments, the isolated population of hypoimmunogenic endothelial cells is expanded before administration. In certain embodiments, the isolated population of hypoimmunogenic endothelial cells is expanded and cryopreserved before administration.

Additional descriptions of endothelial cells for use in the present disclosure can be found in WO2020/018615, the disclosures of which are incorporated herein by reference in their entirety.

### 4. Thyroid cells differentiated from pluripotent stem cells

In some embodiments, the pluripotent stem cells can be differentiated into thyroid progenitor cells and thyroid follicular organoids that can secrete thyroid hormones to address autoimmune thyroiditis. Technologies for differentiating thyroid cells are known in the art. See, e.g., Kurmann et al., Cell Stem Cell, November 5, 2015; 17(5):527-42, the contents of which are incorporated herein by reference in their entirety and specifically for methods and reagents for the generation of thyroid cells from human pluripotent stem cells, as well as transplantation technologies. Differentiation can be determined as is known in the art, generally by assessing the presence of thyroid cell-associated or specific markers or by functional measurement.

### 5. Hepatocytes differentiated from pluripotent stem cells

In some embodiments, the pluripotent stem cells may be differentiated into hepatocytes to address loss of hepatocyte function or cirrhosis of the liver. There are a number of technologies that can be used to differentiate HIP cells into hepatocytes; see, e.g., Pettinato et al., doi: 10.1038/spre32888, Snykers et al., Methods Mol Biol 698:305-314 (2011), Si-Tayeb et al., Hepatology 51:297-305 (2010), and Asgari S, Moslem M, Bagheri-Lankarani K, Pournasr B, Miryounesi M, Baharvand H. Stem Cell Rev Rep. 2013 Aug; 9(4):493-504, all of which are incorporated herein by reference in their entirety and specifically for methods and reagents for differentiation. As is known in the art, differentiation is generally determined by evaluating the presence of hepatocyte-associated and/or specific markers, including but not limited to albumin, alpha fetoprotein, and fibrinogen. Differentiation can also be measured functionally, such as the metabolism of ammonia, LDL storage and uptake, ICG uptake and release, and glycogen storage.

### 6. Islet cells differentiated from pluripotent stem cells

The present disclosure provides pluripotent stem cells that can be differentiated into various islet cell types for subsequent transplantation or implantation into a subject (e.g., a recipient). As will be appreciated by those skilled in the art, the differentiation method depends on the desired cell type using known technologies. Exemplary islet cell types include, but are not limited to, islet progenitor cells, immature islet cells, and mature islet cells. In some embodiments, the islet cells described herein are administered to a subject to treat diabetes.

In some embodiments, the islet cells are derived from the hypoimmunogenic pluripotent cells described herein. Useful methods for differentiating pluripotent stem cells into islet cells are described in, e.g., US9,683,215; US9,157,062; and US8,927,280.

In some embodiments, the hypoimmunogenic pluripotent cells are differentiated into β-like cells or islet organoids for transplantation to address type I diabetes mellitus (T1DM). Cell systems are a promising method for addressing T1DM. See Ellis et al., Nat Rev Gastroenterol Hepatol. October 2017; 14(10):612-628, which is incorporated herein by reference. In addition, Pagbuca et al. report on the successful differentiation of b cells from hiPSCs (Pagbuca et al., Cell, 2014, 159(2):428-39, the contents of which are incorporated herein by reference in their entirety and specifically for the methods and reagents outlined therein for the large-scale production of functional human b cells from human pluripotent stem cells). In addition, Vegas et al. report on the production of human b cells from human pluripotent stem cells followed by encapsulation to avoid immunological rejection by the host (Vegas et al., Nat Med, 2016, 22(3):306-11, the contents of which are incorporated herein by reference in their entirety and specifically for the methods and reagents outlined therein for the large-scale production of functional human b cells from human pluripotent stem cells).

In some embodiments, a method for producing a population of hypoimmunogenic islet cells from a population of hypoimmunogenic pluripotent cells by *in vitro* differentiation comprises: (a) culturing the population of HIP cells in a first culture medium comprising one or more factors selected from insulin-like growth factor (IGF), transforming growth factor (TGF), fibroblast growth factor (EGF), epidermal growth factor (EGF), hepatocyte growth factor (HGF), sonic hedgehog (SHH), vascular endothelial growth factor (VEGF), the transforming growth factor-b (TGF-b) superfamily, bone morphogenetic protein-2 (BMP2), bone morphogenetic protein-7 (BMP7), a GSK inhibitor, an ALK inhibitor, a BMP type 1 receptor inhibitor, and retinoic acid to produce a population of immature islet cells; and (b) culturing the population of immature islet cells in a second culture medium different from the first culture medium to produce a population of hypoimmunogenic islet cells. In some embodiments, the GSK inhibitor is CHIR-99021, a derivative thereof, or a variant thereof. In some instances, the GSK inhibitor is at a concentration ranging from about 2 mM to about 10 mM. In some embodiments, the ALK inhibitor is SB-431542, a derivative thereof, or a variant thereof. In some instances, the ALK inhibitor is at a concentration ranging from about 1 pM to about 10 pM. In some embodiments, the first culture medium and/or the second culture medium does not comprise animal serum.

In some embodiments, the hypoimmunogenic population of islet cells is isolated from non-islet cells. In some embodiments, the isolated population of hypoimmunogenic islet cells is expanded before administration. In certain embodiments, the isolated population of hypoimmunogenic islet cells is expanded and cryopreserved before administration.

As is known in the art, differentiation is generally determined by assessing the presence of b cell-associated or specific markers (including but not limited to insulin). Differentiation can also be measured functionally, e.g., by measuring the metabolism of glucose. See generally Muraro et al., Cell Syst. October 26, 2016; 3(4):385-394.e3, the contents of which are hereby incorporated by reference in their entirety and specifically for the biomarkers outlined therein. Once β cells are produced, they can be transplanted (either as a cell suspension or within a gel matrix as discussed herein) into the portal vein/liver, the omentum, the gastrointestinal mucosa, the bone marrow, a muscle, or a subcutaneous bursa.

Additional descriptions of islet cells, including dopaminergic neurons, for use in the present disclosure can be found in WO2020/018615, the disclosures of which are incorporated herein by reference in their entirety.

### 7. Retinal pigmented epithelium (RPE) cells differentiated from pluripotent stem cells

The present disclosure provides hypoimmunogenic pluripotent cells that can be differentiated into various RPE cell types for subsequent transplantation or implantation into a subject (e.g., a recipient). As will be appreciated by those skilled in the art, the differentiation method depends on the desired cell type using known technologies. Exemplary RPE cell types include, but are not limited to, retinal pigment epithelium (RPE) cells, RPE progenitor cells, immature RPE cells, mature RPE cells, and functional RPE cells.

Useful methods for differentiating pluripotent stem cells into RPE cells are described in, e.g., US9,458,428 and US9,850,463, the disclosures of which are incorporated herein by reference in their entirety, including the specifications. Other methods for producing RPE cells from human induced pluripotent stem cells can be found in, e.g., Lamba et al., PNAS, 2006, 103(34):12769-12774; Melough et al., Stem Cells, 2012, 30(4):673-686; Idelson et al., Cell Stem Cell, 2009, 5(4):396-408; Rowland et al., Journal of Cellular Physiology, 2012, 227(2):457-466; Buchholz et al., Stem Cells Trans Med, 2013, 2(5):384-393; and da Cruz et al., Nat Biotech, 2018, 36:328-337.

In some embodiments, the RPE cells described herein are administered to a subject to treat an ocular disorder selected from wet macular degeneration, dry macular degeneration, juvenile macular degeneration (e.g., Stargardt disease, Best disease, and juvenile retinoschisis), Leber congenital ameurosis, retinitis pigmentosa, retinal detachment, age-related macular degeneration (AMD), early AMD, intermediate AMD, late AMD, and non-neovascular age-related macular degeneration.

Human pluripotent stem cells have been differentiated into RPE cells using the technologies outlined in Kamao et al., Stem Cell Reports 2014:2:205-18, which is incorporated herein by reference in its entirety and specifically for the methods and reagents outlined therein for differentiation technologies and reagents; see also Mandai et al., N Engl J Med, 2017, 376:1038-1046, the contents of which are incorporated herein in their entirety for an understanding of technologies for generating sheets of RPE cells and transplantation into patients. Differentiation can be determined as is known in the art, generally by assessing the presence of RPE-associated and/or specific markers or by functional measurement. See, e.g., Kamao et al., Stem Cell Reports, 2014, 2(2):205-18, the contents of which are incorporated herein by reference in their entirety and specifically for the markers outlined in the first paragraph of the results section.

In some embodiments, a method for producing a population of hypoimmunogenic retinal pigment epithelium (RPE) cells from a population of hypoimmunogenic pluripotent cells by *in vitro* differentiation comprises: (a) culturing the population of hypoimmunogenic pluripotent cells in a first culture medium comprising a factor selected from activin A, bFGF, BMP4/7, DKKl, IGFl, noggin, a BMP inhibitor, an ALK inhibitor, a ROCK inhibitor, and a VEGFR inhibitor to produce a population of pre-RPE cells; and (b) culturing the population of pre-RPE cells in a second culture medium different from the first culture medium to produce the population of hypoimmunogenic RPE cells. In some embodiments, the ALK inhibitor is SB-431542, a derivative thereof, or a variant thereof. In some instances, the ALK inhibitor is at a concentration ranging from about 2 mM to about 10 mM. In some embodiments, the ROCK inhibitor is Y-27632, a derivative thereof, or a variant thereof. In some instances, the ROCK inhibitor is at a concentration ranging from about 1 pM to about 10 pM. In some embodiments, the first culture medium and/or the second culture medium does not comprise animal serum.

Differentiation can be determined as is known in the art, generally by assessing the presence of RPE-associated and/or specific markers or by functional measurement. See, e.g., Kamao et al., Stem Cell Reports, 2014, 2(2):205-18, the contents of which are incorporated herein by reference in their entirety and specifically for the results section.

Additional descriptions of RPE cells for use in the present disclosure can be found in WO2020/018615, the disclosures of which are incorporated herein by reference in their entirety.

### 8. NK cells differentiated from pluripotent stem cells

The present disclosure provides hypoimmunogenic pluripotent cells that can be differentiated into natural killer (NK) cells. In some embodiments, a method for producing a population of natural killer (NK) cells from a population of hypoimmunogenic pluripotent cells by *in vitro* differentiation comprises: (a) culturing a population of stem cells in a first culture medium comprising a ROCK inhibitor under conditions sufficient for the formation of an aggregate; (b) culturing the aggregate in a second culture medium comprising BMP-4; (c) culturing the aggregate in a third culture medium comprising BMP-4, FGF2, a WNT pathway activator, and activin A; (d) culturing the aggregate in a fourth culture medium comprising FGF2, VEGF, TPO, SCF, IL-3, FLT3L, WNTC-59, and an activin/nodal inhibitor to form a population of cells comprising hematopoietic stem and progenitor cells (HSPCs); (e) culturing the population of cells in a fifth culture medium comprising FGF2, VEGF, TPO, SCF, IL-3, and FLT3L; (f) culturing the population of cells in a sixth culture medium comprising IL-3, IL-7, FLT3L, IL-15, and SCF; and (g) culturing the population of cells in a seventh culture medium comprising IL-7, FLT3L, IL-15, and SCF for a period of time sufficient for the production of NK cells.

In some embodiments, the first culture medium comprises 10 µM ROCK inhibitor. In some embodiments, the second culture medium comprises 30 ng/mL BMP-4. In some embodiments, the second culture medium comprises 30 ng/mL BMP-4 and 10 µM ROCK inhibitor. In some embodiments, the third culture medium comprises 30 ng/mL BMP-4, 100 ng/mL FGF2, 6 µM CHIR-99021, and 2.5-5 ng/mL activin A. In some embodiments, the third culture medium comprises 30 ng/mL BMP-4, 100 ng/mL FGF2, 7 µM CHIR-99021, and 2.5-5 ng/mL activin A. In some embodiments, the fourth and fifth culture media comprise 20 ng/mL FGF, 20 ng/mL VEGF, 20 ng/mL TPO, 100 ng/mL SCF, 40 ng/mL IL-3, and 10-20 ng/mL FLT3L. In some embodiments, the fourth culture medium further comprises 2 µM WNT C-59 and 5 µM SB-431542. In some examples, the fourth culture medium further comprises 5 µM SB-431542. In some embodiments, the fourth culture medium does not comprise WNT C-59. In some embodiments, the sixth and seventh culture media comprise 20 ng/mL IL-7, 10-20 ng/mL FLT3L, 10-20 ng/mL IL-15, and 20 ng/mL SCF. In some embodiments, the sixth culture medium comprises 5 ng/mL IL-3. In some embodiments, the eighth culture medium comprises IL-7, FLT3L, IL-15, SCF, and nicotinamide.

Additional descriptions of NK cells for use in the present disclosure can be found in US20220169700A1, the disclosures of which are incorporated herein by reference in their entirety.

For therapeutic application, cells prepared according to the disclosed methods can generally be provided in the form of a pharmaceutical composition comprising an isotonic excipient, and are prepared under conditions sufficiently sterile for human administration. For general principles of pharmaceutical formulations of cell compositions, see "Cell Therapy: Stem Cell Transplantation, Gene Therapy, and Cellular Immunotherapy", Morstyn & Sheridan eds, Cambridge University Press, 1996; and "Hematopoietic Stem Cell Therapy", E. D. Ball, J. Lister & P. Law, Churchill Livingstone, 2000. The cells can be packaged in a device or container suitable for distribution or clinical use.

### Beneficial Effects

The pluripotent stem cells of the present disclosure and hypoimmunogenic pluripotent stem cells prepared by the methods of the present disclosure can exhibit excellent effects, for example, but not limited to: (1) having good self-renewal and differentiation abilities, (2) being able to escape ADCC killing, (3) being able to escape NK cell killing, (4) being able to escape PBMC killing, (5) being able to escape MAC cell killing, and/or (6) the differentiated cells being also able to escape NK cell killing, T cell killing, MAC cell killing, or ADCC killing, thereby showing excellent application potential.

### Examples

The present disclosure is further illustrated with reference to the following examples. It will be appreciated that these examples are merely exemplary and are not intended to limit the present disclosure. The following materials and instruments were commercially available or prepared according to methods well known in the art. The following experiments were conducted according to the manufacturer's instructions or according to methods and steps well known in the art.

Where specific conditions are not indicated in experimental methods in the following examples, conventional conditions such as those described in Sambrook et al., Molecular Cloning: A Laboratory Manual (New York: Cold Spring Harbor Laboratory Press, 1989) or conditions recommended by the manufacturer are generally followed.

Unless defined otherwise or clearly indicated by the context, all technical and scientific terms in the present disclosure have the same meanings as commonly understood by those of ordinary skill in the art to which the present disclosure pertains.

### Example 1. Construction of B2M/CIITA Double Knockout (DKO) Cell Lines 1.1. Construction of B2M/CIITA double knockout (DKO) cell lines

In the following example, human induced pluripotent stem cells (iPSCs) were selected for the construction of the target cell line. The human induced pluripotent stem cells (iPSCs) used were prepared from human peripheral blood cells using a CTS^{™} CytoTune^{™}-iPS 2.1 Sendai Reprogramming Kit (Cat. No. A34546) according to the manufacturer's instructions. Human pluripotent stem cell line H1 (Wicell, WA01) or H9 (Wicell, WA09) could also be used to construct the target cell line (human pluripotent stem cell line H1 or H9 is also referred to as hESCs). The cell culture and gene knockout reagents used are shown in Table 1.

**Table 1. Cell culture and gene knockout reagents**

| Reagent name | Company |
|---|---|
| mTeSR^{™} Plus | Stemcell |
| TrypLE^{™} Express enzyme (1×), phenol red-free | ThermoFisher |
| Matrigel^{®} hESC-Qualified Matrix, *LDEV-Free, 5 mL | Corning^{®} |
| DMEM/F-12, GlutaMAX^{™} supplement | ThermoFisher |
| Y-27632 | Stemcell |
| TrueCut^{™} Cas9 Protein v2 | ThermoFisher |

β-2-microglobulin (B2M) was knocked out from the endoplasmic reticulum using CRISPR/CAS9 to prevent the formation of functional MHC-I molecules on the cell surface, thereby enabling the cells to escape allogeneic CD8⁺ T cell killing. To enable the cells to escape CD4+ T cell killing, CIITA, a positive regulatory factor of MHC-II gene transcription, was knocked out to reduce the expression of MHC class II molecules. In the present disclosure, the double knockout (DKO) pluripotent stem cells of the H1 cell line are also referred to as DKO cells or H1 cell-derived DKO cells, and the DKO pluripotent stem cells of the iPSC cell line are referred to as iPSC-DKO cells or iPSC-derived DKO cells.

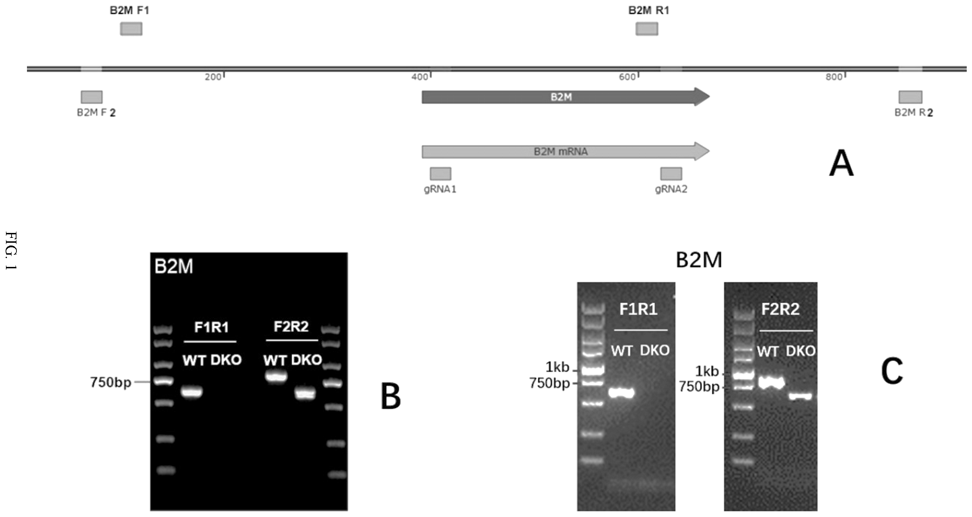

The CRISPR/CAS9 gene knockout strategy for B2M and the gRNA sequences and identification primers used are shown in FIG. 1 and Table 2. B2M-gRNA1 and B2M-gRNA2 (EasyEdit sgRNA, GenScript) were used to perform direct two-end knockout of the exons of B2M, and genome sequence knockout validations were then performed using two pairs of PCR primers: B2M-F1/R1 and B2M-F2/R2, respectively. A of FIG. 1 shows the B2M gene knockout strategy for the B2M/CIITA double knockout (DKO) cell line, B of FIG. 1 shows the results of the validation of B2M gene knockout from the H1 cell line by PCR, and C of FIG. 1 shows the results of the validation of B2M gene knockout from the iPSC cell line by PCR.

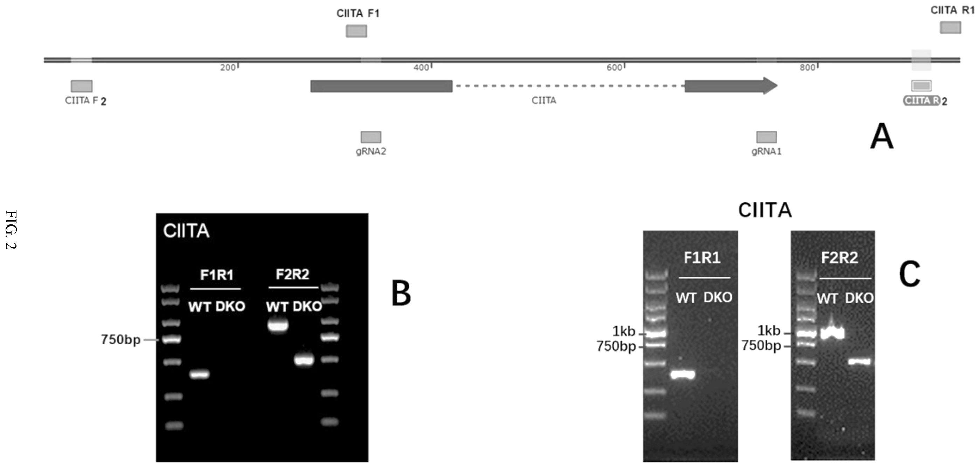

In addition, the CRISPR/CAS9 gene knockout strategy for CIITA and the gRNA sequences and identification primers used are shown in FIG. 2 and Table 2. CIITA-gRNA1 and CIITA-gRNA2 (EasyEdit sgRNA, GenScript) were used to perform direct two-end knockout of the exons of CIITA, and genome sequence knockout validations were then performed using two pairs of PCR primers: CIITA-F1/R1 and CIITA-F2/R2, respectively. A of FIG. 2 shows the CIITA gene knockout strategy for the B2M/CIITA double knockout (DKO) cell line, B of FIG. 2 shows the results of the validation of CIITA gene knockout from the H1 cell line by PCR, and C of FIG. 2 shows the results of the validation of CIITA gene knockout from the iPSC cell line by PCR.

**Table 2. gRNA sequences and identification primers**

| **gRNA sequence** | **Specific sequence** | |
|---|---|---|
| B2M-gRNA1 (SEQ ID NO: 7) | CGTGAGTAAACCTGAATCTT | |
| B2M-gRNA2 (SEQ ID NO: 8) | AGTCACATGGTTCACACGGC | |
| CIITA-gRNA1 (SEQ ID NO: 9) | GATATTGGCATAAGCCTCCC | |
| CIITA-gRNA2 (SEQ ID NO: 10) | CATCGCTGTTAAGAAGCTCC | |

| **Identification primer** | **Specific sequence** | **Description** |
|---|---|---|
| B2M-F1 (SEQ ID NO: 11) | TGGGGCCAAATCATGTAGACTC | B2M-F2+B2M-R2 = 608 bp |
| B2M-R1 (SEQ ID NO: 12) | TCAGTGGGGGTGAATTCAGTGT | After knockout: no band |
| B2M-F2 (SEQ ID NO: 13) | CAGAAGTCCTTGAGAGCCTCC | B2M-F2+B2M-R2 = 812 bp |
| B2M-R2 (SEQ ID NO: 14) | TGTGCATCAGTATCTCAGCAGG | After knockout: 569 bp |
| CIITA-F1 (SEQ ID NO: 15) | CTGTGCCTCTACCACTTCTATG | CIITA-F1+CIITA-R1 = 368 bp |
| CIITA-R1 (SEQ ID NO: 16) | CCTTCCATGTCACACAACAGCC | After knockout: no band |
| CIITA-F2 (SEQ ID NO: 17) | TGGAATCCACACTTTCCAGTTC | CIITA-F2+CIITA-R2 = 889 bp |
| CIITA-R2 (SEQ ID NO: 18) | TGGAGTCTCCGTTCCTCCAG | After knockout: 459 bp |

The specific procedures were as follows:
1) Human induced pluripotent stem cells were routinely cultured in an mTeSR1 culture medium supplemented with Y-27632 on a Matrigel-coated 6-well plate until the density was 80%. The cells were digested with TrypLE, then neutralized with DMEM/F12, and counted. 2 × 10⁶ cells were pipetted into an EP tube and centrifuged, and the supernatant was then discarded.
2) According to a 100 µL electroporation system of a Neon transfection system (ThermoFisher), 15 µg of TrueCut^{™} Cas9 Protein and 3 µg of gRNA (B2M-gRNA1 + B2M-gRNA2 + CIITA-gRNA1 + CIITA-gRNA2) were added to form a ribonucleoprotein complex (RNP) system. After thorough mixing, the system was left to stand at room temperature for 20 min.
3) The cells were resuspended in 100 µL of the RNP electroporation system, and electroporation was performed using the Neon transfection system with the following parameters: 1200 V, 30 ms, and 1 pause. A pre-heated culture medium was rapidly added to the cells after electroporation, and the cells were then seeded evenly in a 6-well plate with one well coated with Matrigel.
4) The culture medium was replaced daily with a fresh mTeSR1 culture medium. When single-cell colonies became visible, individual clones were picked and transferred into a 48-well plate. After clonal expansion, genome samples were collected for PCR analysis to determine gene editing outcomes. The PCR results are shown in FIGs. 1 and 2. PCR-positive clones were sent for Sanger sequencing at a company for further validation.
5) The B2M/CIITA double allele knockout (DKO) clones with positive identification results were expanded and cryopreserved.

### 1.2. Assessment of expression of B2M and CIITA at RNA level in DKO

Total RNA was extracted from H1 cells and the derived DKO cells using the FastPure Cell/Tissue Total RNA Isolation Kit V2 (Vazyme, RC112-01). Then, the RNA was reverse-transcribed into cDNA using HiScript III RT SuperMix for qPCR (Vazyme, R323-01) according to the manufacturer's instructions.

The expression of B2M and CIITA at an RNA level in DKO cells derived from B2M/CIITA double allele knockout clone H1 cells was assessed by qPCR using the primers below.
B2M-F (SEQ ID NO: 19): AAGATGAGTATGCCTGCCGT
B2M-R (SEQ ID NO: 20): ATGCGGCATCTTCAAACCTC
CIITA-F (SEQ ID NO: 21): CCTGGAGCTTCTTAACAGCGA
CIITA-R (SEQ ID NO: 22): TGTGTCGGGTTCTGAGTAGAG

A Roche 480II instrument was used. The reaction system was as follows:
Pre-incubation, 95 °C, 30 s.
Amplification, 95 °C for 10 s, 60 °C for 30 s, 40 cycles.
Default Melting curve and Cooling programs were used.

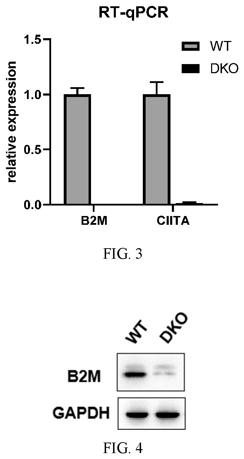

The qPCR results are shown in FIG. 3. The expression of B2M and CIITA at an RNA level was nearly absent in the B2M/CIITA double allele knockout (DKO) clones compared to the untreated wild-type human pluripotent stem cells, indicating that B2M and CIITA had been successfully knocked out.

### 1.3. Assessment of expression of B2M at protein level in DKO

A Western blot was used to assess the expression of B2M at a protein level in DKO cells derived from B2M/CIITA double allele knockout clone H1 cells (B2M antibody Cat. No. ab75853; internal reference antibody GAPDH Cat. No. ab181602; both purchased from Abcam).

The Western blot results are shown in FIG. 4. The B2M protein level in the DKO cells derived from H1 cells was significantly reduced compared to the wild-type human pluripotent stem cells, indicating that the B2M protein had been successfully knocked out.

### 1.4. Detection of HLA class I/II molecules of DKO

WT and DKO cells were stimulated with IFN-γ (PeproTech, Cat#300-02) to detect HLA class I/II molecules on the surface of the H1 cell (WT) line and the iPSC (iPSC-WT) cell line, as well as DKO cells derived from the two cell lines (DKO cells and iPSC-DKO cells). The specific method was as follows:
The cells were plated. The next day, at the time of refreshing the culture medium, a culture medium containing IFN-γ was added to the cells. After 48 h of treatment, the cells were digested and then assayed for HLA-I/II expression using a flow cytometer (Agilent Technologies, NovoCyte).

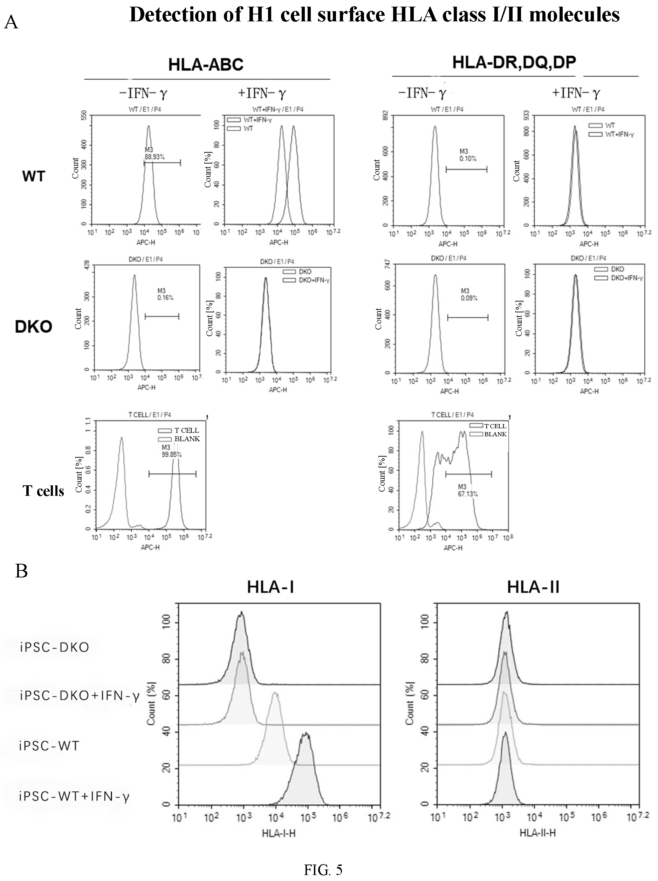

The flow cytometry assay results are shown in FIG. 5. A of FIG. 5 shows the assay results for H1 cells and DKO cells, and B of FIG. 5 shows the assay results for iPSCs and iPSC-DKO cells. The detection of HLA class I molecules (HLA-ABC) is shown in the left parts of both panels, and the detection of HLA class II molecules (HLA-DR, DQ, and DP) is shown in their right parts. T cells were the positive control of the assay. It was observed that the DKO cells derived from the B2M/CIITA double allele knockout clone H1 and iPSC cell lines (DKO cells and iPSC-DKO cells) were incapable of expressing HLA class I/II molecules in response to IFN-γ stimulation, demonstrating that the HLA-I and HLA-II functions of the DKO cells and iPSC-DKO cells were reduced.

### 1.5. Karyotype test of DKO

The obtained B2M/CIITA double allele knockout (DKO) positive clones of H1 cells were subjected to a karyotype test. The specific method was as follows:
Chromosome specimens fixed on slides were treated with trypsin and then stained with Giemsa stain. Based on characteristics such as chromosome length, centromere position, ratio of long arm to short arm, presence or absence of satellites, etc., metaphase chromosomes were analyzed for chromosome number and morphological structure to determine whether their karyotype was consistent with the normal karyotype.

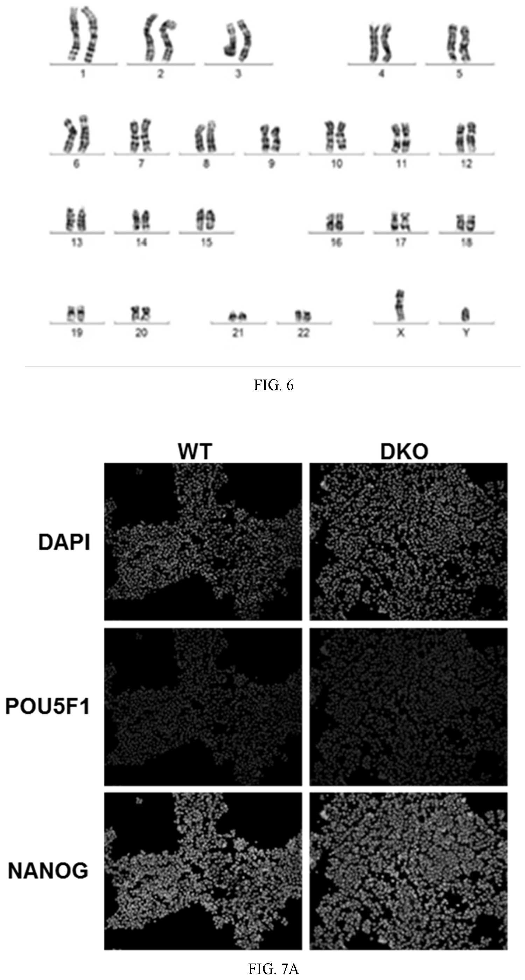

The results of the karyotype test are shown in FIG. 6. The karyotype of the DKO cells derived from H1 cells was normal-it was not significantly different from the normal karyotype.

### Example 2. Stemness and Immune Function of DKO Cell Line

In this example, whether the stemness and immune function of pluripotent stem cells changed after B2M/CIITA double allele knockout was further determined.

### 2.1. Expression of pluripotency genes in DKO cells

The protein levels of pluripotency genes in WT cells of the iPSC and H1 cell lines (WT cells and iPSC-WT cells) and DKO cells derived from the cell lines (DKO cells and iPSC-DKO cells) were determined by immunofluorescence, the expression of pluripotency genes at an RNA level in H1 cells (WT cells) and derived DKO cells (DKO cells) was assessed by RT-qPCR, and the expression of the proteins corresponding to pluripotency genes on the surface of H1 cells (WT cells) and iPSC-WT cells and DKO cells (DKO cells and iPSC-DKO cells) was assessed by flow cytometry. The specific methods were as follows:
Immunofluorescence assay: WT or DKO cells were plated onto a 12-well plate. After the cells grew to a density of 60-80%, the culture medium was discarded using a pipette, and 4% paraformaldehyde was added for fixation. After cell membrane permeabilization, the cells were incubated with primary antibodies of POU5F1 and NANOG, as well as other pluripotency gene proteins, at 4 °C overnight, washed to remove the primary antibodies, and then incubated with fluorescently labeled secondary antibodies at room temperature. Subsequently, photos were taken using a fluorescence microscope (Nikon Ts2R-FL).

### RT-qPCR assay:

A Roche 480II instrument was used. The reaction system was as follows:
Pre-incubation, 95 °C, 30 s.
Amplification: 95 °C for 10 s, 60 °C for 30 s, 40 cycles.
Default Melting curve and Cooling programs were used.
Mesenchymal stem cells (MSCs) were used as a negative control for pluripotency gene expression.

### Flow cytometry:

Cells were collected and then incubated with antibodies in an EP tube in a dark place at 4 °C for 30 min. Then, an appropriate fluorescence acquisition channel was selected based on antibody information, and fluorescence was measured on the instrument. All antibodies were from BD Biosciences.

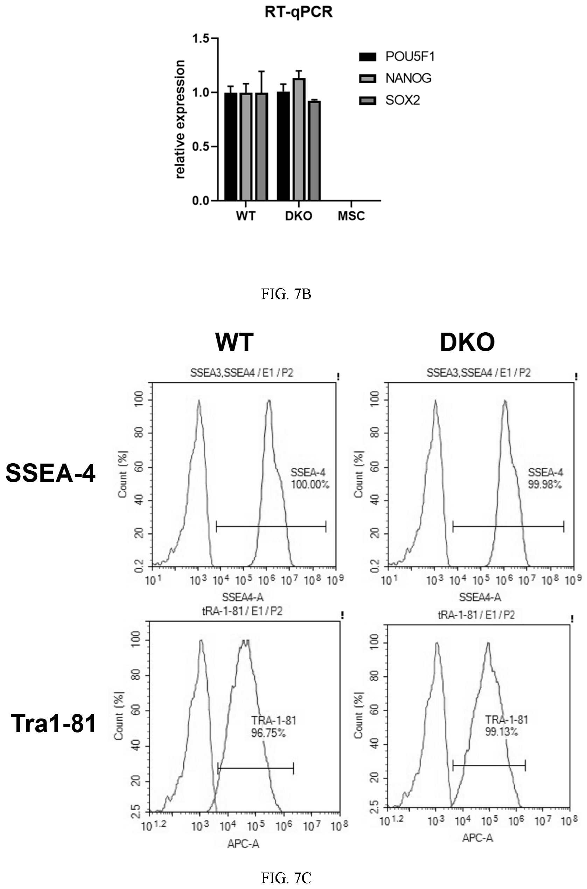

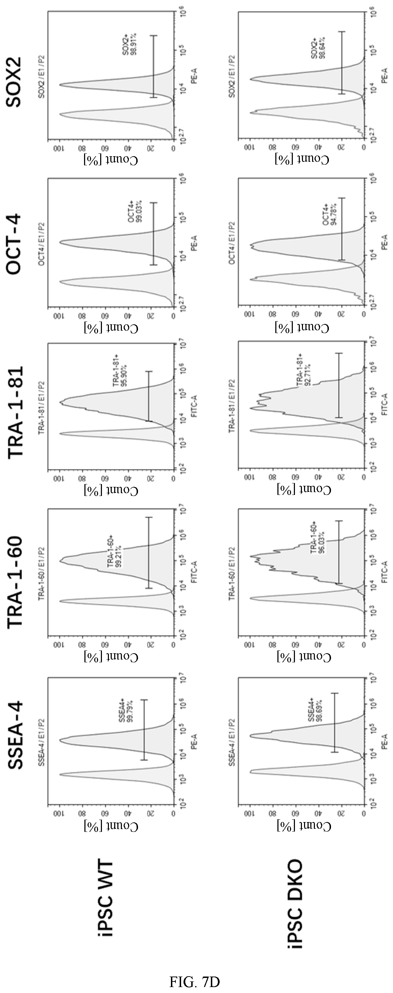

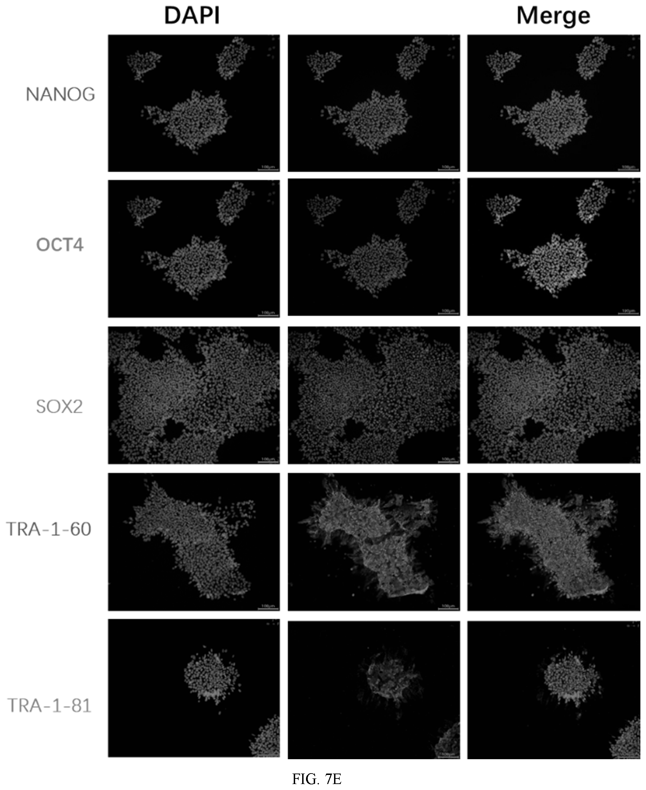

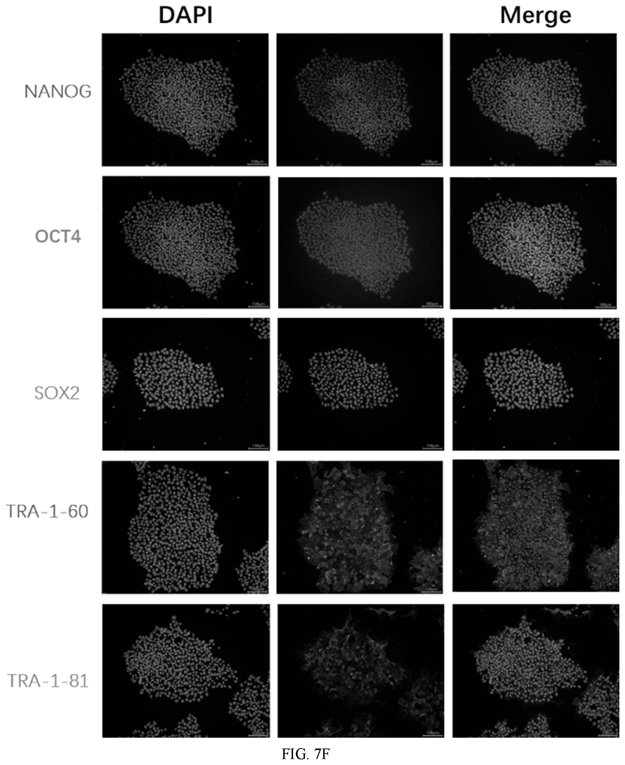

The immunofluorescence assay results are shown in FIG. 7A: both the H1 cells (WT) and the H1 cell-derived DKO cells expressed the POU5F1 and NANOG pluripotency genes at a protein level, and the expression of the POU5F1 and NANOG pluripotency genes in the H1 cell-derived DKO cells was not significantly different from that in the WT cells. The RT-qPCR assay results are shown in FIG. 7B: the H1 cells (WT) and the H1 cell-derived DKO cells expressed the POU5F1, NANOG, and SOX2 pluripotency genes at an RNA level, and the expression of the POU5F1, NANOG, and SOX2 pluripotency genes in the H1 cell-derived DKO cells was not significantly different from that in the H1 cells (WT). The flow cytometry assay results are shown in FIG. 7C: both the H1 cells (WT) and the H1 cell-derived DKO cells highly expressed the proteins corresponding to the pluripotency genes SSEA-4 (WT 100% vs. DKO 99.98%) and Tra1-81 (WT 96.75% vs. DKO 99.13%) on their surface. FIG. 7D shows the results of the assessment of the expression of the SSEA-4, TRA-1-60, TRA-1-81, OCT-4, and SOX2 pluripotency genes on the surface of iPSC-WT and iPSC-DKO cells by flow cytometry: both the iPSC-WT cells and the iPSC-derived DKO cells highly expressed the proteins corresponding to the pluripotency genes on their surface. FIG. 7E shows the results of the determination of the protein levels of the TRA-1-60, TRA-1-81, OCT-4, SOX2, and NANOG pluripotency genes in iPSC-WT cells by immunofluorescence: the iPSC-WT cells highly expressed the proteins corresponding to the pluripotency genes on their surface. FIG. 7F shows the results of the determination of the protein levels of the TRA-1-60, TRA-1-81, OCT-4, SOX2, and NANOG pluripotency genes in iPSC-DKO cells by immunofluorescence: the iPSC-derived DKO cells highly expressed the proteins corresponding to the pluripotency genes on their surface.

### 2.2. Differentiation ability of DKO cells

In this example, the differentiation ability of DKO cells was assessed. The specific method was as follows: Immunodeficient mice (SCID Beige, Charles River) were each subcutaneously injected with 100 µL of a suspension containing 5 × 10⁵ DKO cells. After the teratoma volume was greater than 1.5 cm³, the teratomas were collected and subjected to paraffin sectioning and hematoxylin and eosin staining.

The staining results show that the H1 cell-derived B2M/CIITA double allele knockout (DKO) cells could form teratomas *in vivo* and differentiate into cells of three germ layers (an endoderm, a mesoderm, and an ectoderm), indicating that the H1 cell-derived DKO cells have the ability to normally differentiate into the three germ layers.

### 2.3. Immune function of DKO cells

T cell and NK cell killing experiments were conducted using an xCELLigence RTCA instrument to determine changes in the immune function of H1 cells, iPSCs, and derived DKO cells. The reagents used in the killing experiments are shown in Table 3.

The specific method was as follows: The same number of cells of the WT and DKO cell lines was resuspended in an Essential 8 culture medium containing human IL-2 and seeded in 96-well E-plates coated with Matrigel, and activated T cells (XC11228, purchased from SAILYBIO) or NK cells (XC11013, purchased from SAILYBIO) were added for a killing assay. The T cells were assayed by flow cytometry for CD3, CD4, and CD8 before use, and the NK cells were assayed by flow cytometry for CD16 and CD56 before use, so as to ensure the functioning of the T cells and NK cells used. RTCA data were analyzed using xCELLigence software to calculate the killing rate and escape function.

**Table 3. The reagents used in killing experiments**

| **Name** | **Catalog number (Cat No.)** | **Specification** | **Manufacturer** |
|---|---|---|---|
| PE anti-human CD4 | 980804 | 500 µL/tube | Biolegend |
| APC anti-human CD8 | 980904 | 500 µL/tube | Biolegend |
| FITC anti-human CD3 | 300440 | 500 times | Biolegend |
| APC anti-human CD16 | 301012 | 100 times | Biolegend |
| PE anti-human CD56 (NCAM) | 318306 | 100 times | Biolegend |
| Human IL-2 | 202-1L-050/CF | 50 µg | R&D |
| Y-27632 2HCl | S1049 | 5 mg | Selleck |
| Matrigel | 354277 | 5 mL | Gibco |
| Essential 8^{™} culture medium | A1517001 | 500 mL | Gibco |
| E-Plate VIEW 96 PET | 300601030 | 6 plates/pack | Agilent |

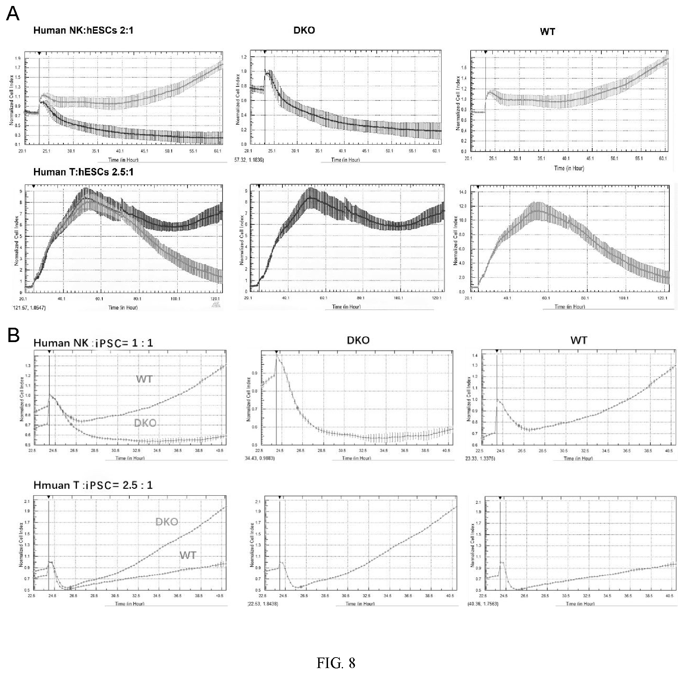

The RTCA results are shown in FIG. 8. The WT cells escaped NK cell killing due to the expression of HLA-I, but could be killed by T cells. The DKO cells could escape T cell killing but were more sensitive to NK cell killing. The upper three plots of A of FIG. 8 show the results of the determination of the killing rates of NK cells against H1 cells (WT) and H1 cell-derived DKO cells by RTCA, i.e., the results of the assessment of the immune escape function of H1 cells (WT) and H1 cell-derived DKO cells against NK cells. The three plots in the second row of A of FIG. 8 show the results of the determination of the killing rates of T cells against H1 cells (WT) and H1 cell-derived DKO cells by RTCA, i.e., the results of the assessment of the immune escape function of WT cells and H1 cell-derived DKO cells against T cells. The upper three plots of B of FIG. 8 show the results of the determination of the killing rates of NK cells against iPSC-WT and iPSC-DKO cells by RTCA, i.e., the results of the assessment of the immune escape function of iPSC-WT and iPSC-DKO cells against NK cells. The three plots in the second row of B of FIG. 8 show the results of the determination of the killing rates of T cells against iPSC-WT and iPSC-DKO cells by RTCA, i.e., the results of the assessment of the immune escape function of iPSC-WT and iPSC-DKO cells against T cells.

### Example 3. Construction and Immune Function Validation of DKO+CD47 Cell Line

### 3.1. Construction of DKO+CD47 cell line of H1 cells

In this example, a lentiviral vector was used to overexpress CD47 (NM_198793) in the DKO cells obtained in Example 1. The amino acid sequence of CD47 is set forth in SEQ ID NO: 5.

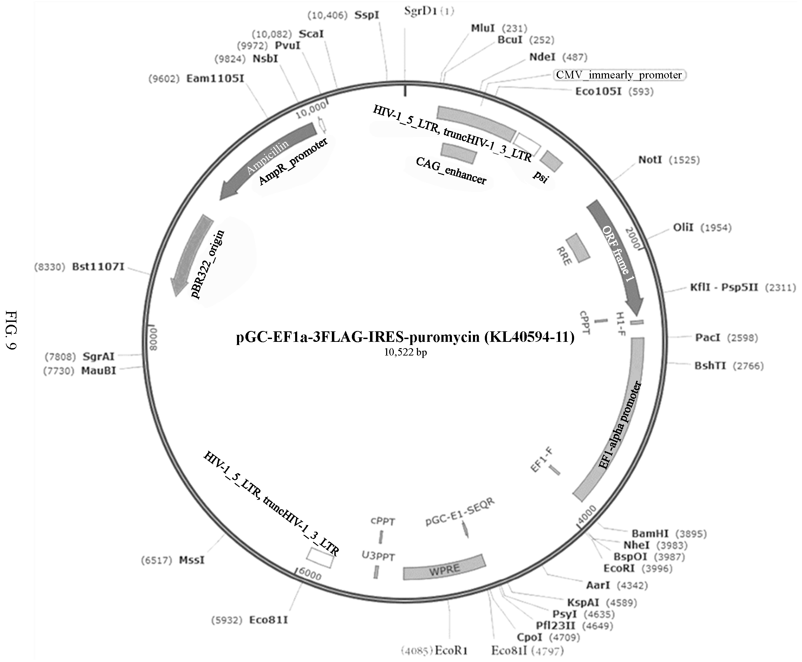

The nucleic acid sequence encoding the CD47 protein (SEQ ID NO: 6) was constructed in a lentiviral vector (pGC-EF1a) driven by EF1a and carrying a puromycin screening marker. The structure of the pGC-EF1a vector is shown in FIG. 9. The specific procedure was as follows:
The lentiviral vector was digested with *BamH*I/*Nhe*I, and the nucleic acid sequence encoding the CD47 protein (SEQ ID NO: 6) was ligated to the lentiviral vector. After the ligation was successful, Sanger sequencing was used to determine whether the sequence was correctly inserted, and viral packaging was performed. The lentiviral vector was transfected into the DKO cells constructed in Example 1. After 24 h, the culture medium was refreshed. After 48 h, the culture medium was replaced with a culture medium containing puromycin for screening.

The constructed stably transfected DKO+CD47 cells were assayed by flow cytometry (the CD47 antibody was purchased from FACS: Biolegend, Cat. No. 323108) and qPCR. The qPCR primers were as follows:
CD47-F (SEQ ID NO: 26): AGAAGGTGAAACGATCATCGAGC;
CD47-R (SEQ ID NO: 27): CTCATCCATACCACCGGATCT.

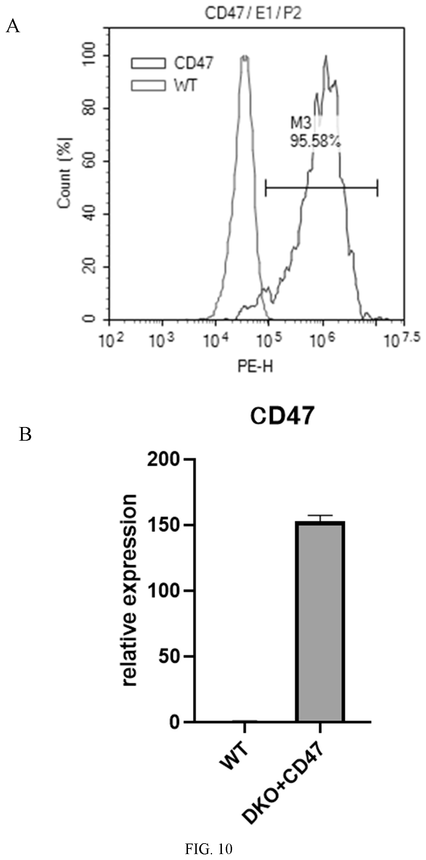

The assay results are shown in A and B of FIG. 10. In the constructed DKO+CD47 cell line, the expression level of CD47 was significantly higher than that in WT cells. Moreover, after validation, the DKO+CD47 cell line was subjected to cell expansion and subsequent functional assays.

### 3.2. Validation of immune function of DKO+CD47

Whether the overexpression DKO+CD47 cell strain could successfully escape NK cell killing while escaping T cell killing was determined by RTCA. See Example 2.3 for the specific method.

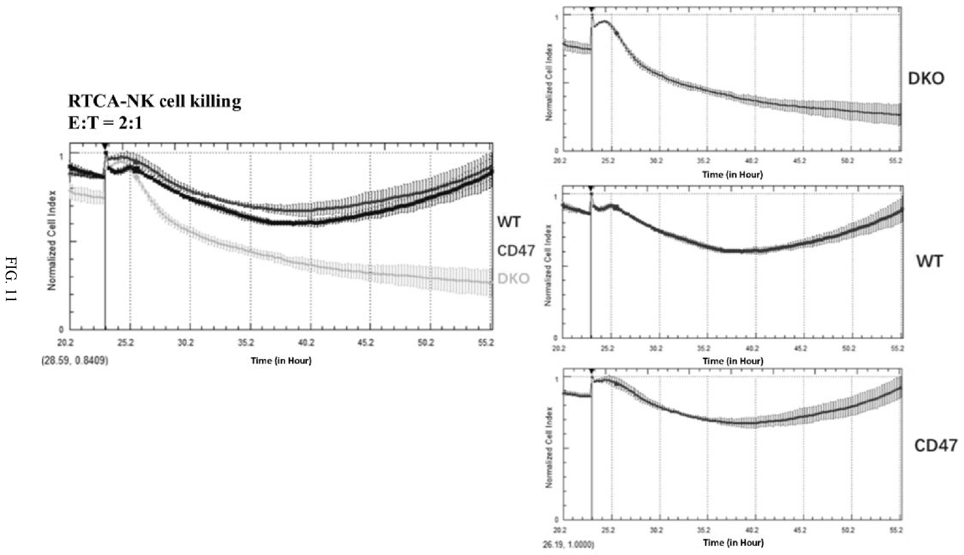

The RTCA is shown in FIG. 11. NK cells could effectively kill the DKO cells, while the WT and DKO+CD47 overexpression cells could escape NK cell killing.

### Example 4. Construction and Stemness and Differentiation Ability Assays of DKO+MCL1 Cell Line

### 4.1. Construction and overexpression assay of iPSC-DKO+MCL1 cell line

The nucleic acid sequence encoding the MCL1 protein (the amino acid sequence of the MCL1 protein is set forth in SEQ ID NO: 3) was directly synthesized.

Amino acid sequence of MCL1 (SEQ ID NO: 3):

MFGLKRNAVIGLNLYCGGAGLGAGSGGATRPGGRLLATEKEASARREIGGGEAGAVIGGSAGASPP
STLTPDSRRVARPPPIGAEVPDVTATPARLLFFAPTRRAAPLEEMEAPAADAIMSPEEELDGYEPEPL
GKRPAVLPLLELVGESGNNTSTDGSLPSTPPPAEEEEDELYRQSLEIISRYLREQATGAKDTKPMGRS
GATSRKALETLRRVGDGVQRNHETAFQGMLRKLDIKNEDDVKSLSRVMIHVFSDGVTNWGRIVTL
ISFGAFVAKHLKTINQESCIEPLAESITDVLVRTKRDWLVKQRGWDGFVEFFHVEDLEGGIRNVLLA
FAGVAGVGAGLAYLIR

Nucleic acid sequence of MCL1 (SEQ ID NO: 25):

atgtttggcctcaaaagaaacgcggtaatcggactcaacctctactgtgggggggccggcttgggggccggcagcggcggcgccacccgcccgggag
ggcgacttttggctacggagaaggaggcctcggcccggcgagagatagggggaggggaggccggcgcggtgattggcggaagcgccggcgcaagc
cccccgtccaccctcacgccagactcccggagggtcgcgcggccgccgcccattggcgccgaggtccccgacgtcaccgcgacccccgcgaggctgc
ttttcttcgcgcccacccgccgcgcggcgccgcttgaggagatggaagccccggccgctgacgccatcatgtcgcccgaagaggagctggacgggtac
gagccggagcctctcgggaagcggccggctgtcctgccgctgctggagttggtcggggaatctggtaataacaccagtacggacgggtcactaccctcg
acgccgccgccagcagaggaggaggaggacgagttgtaccggcagtcgctggagattatctctcggtaccttcgggagcaggccaccggcgccaagg
acacaaagccaatgggcaggtctggggccaccagcaggaaggcgctggagaccttacgacgggttggggatggcgtgcagcgcaaccacgagacgg
ccttccaaggcatgcttcggaaactggacatcaaaaacgaagacgatgtgaaatcgttgtctcgagtgatgatccatgttttcagcgacggcgtaacaaactg
gggcaggattgtgactctcatttcttttggtgcctttgtggctaaacacttgaagaccataaaccaagaaagctgcatcgaaccattagcagaaagtatcacag

acgttctcgtaaggacaaaacgggactggctagttaaacaaagaggctgggatgggtttgtggagttcttccatgtagaggacctagaaggtggcatcagg
aatgtgctgctggcttttgcaggtgttgctggagtaggagctggtttggcatatctaataagatag

As described in Example 3.1, the nucleic acid sequence encoding the MCL1 protein was constructed in a lentiviral vector (pGC-EF1a) driven by EF1a and carrying a puromycin screening marker. The structure of the pGC-EF1a vector is shown in FIG. 9.The specific procedure was as follows: The vector was digested with *BamH*I/*Nhe*I, and the nucleic acid sequence of MCL1 synthesized above was ligated to the lentiviral vector. After the ligation was successful, Sanger sequencing was used to determine whether the sequence was correctly inserted, and viral packaging was performed. The lentiviral vector was transfected into the iPSC-DKO cells constructed in Example 1. After 24 h, the culture medium was refreshed. After 48 h, the culture medium was replaced with a culture medium containing puromycin for screening. The cell strain obtained by screening was designated iPSC-DKO+MCL1 cells.

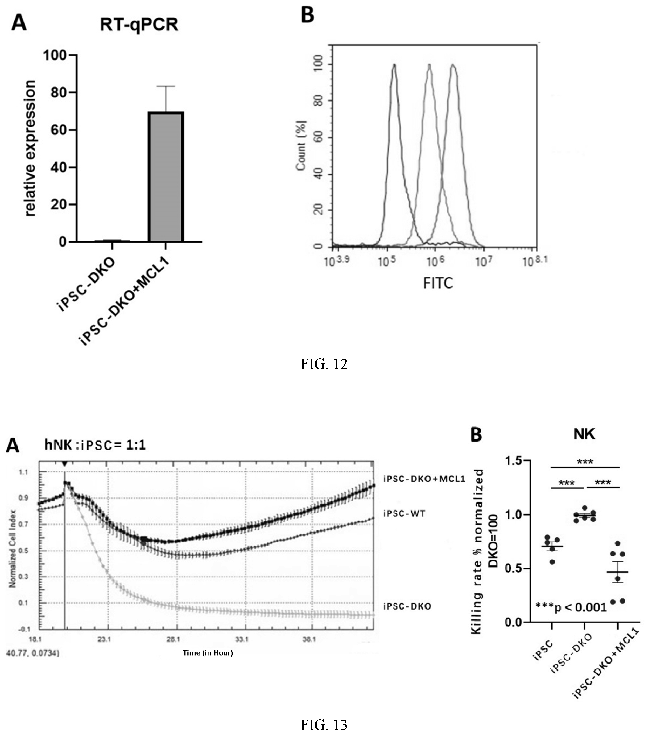

The constructed iPSC-DKO+MCL1 cell line was subjected to an overexpression assay. The overexpression level of the mRNA was determined by qRT-PCR, with iPSC-WT cells as a negative control. The primers used are shown in Table 4. The overexpression level of the protein was determined by FACS, with iPSC-WT cells as negative control cells (the middle peak in B of FIG. 12), and an isotype as a detection signal negative control (the left peak in B of FIG. 12).

**Table 4. Primers for qPCR detection of MCL1**

| **Primer sequence** | **Specific sequence** |
|---|---|
| MCL1 F1 (SEQ ID NO: 23) | GCTTCGGAAACTGGACATCA |
| MCL1 R1 (SEQ ID NO: 24) | GGAAGAACTCCACAAACCCA |

The results are shown in A and B of FIG. 12. The constructed iPSC-DKO+MCL1 cell line (the right peaks in A and B of FIG. 12) exhibited high expression of MCL1.

### Example 5. Immune Escape Function of iPSC-Derived DKO+MCL1 Cell Line

To validate the escape function of iPSC-derived DKO+MCL1 cells against different immune cells, experiments were conducted using NK cells, ADCC (antibody-dependent cellular cytotoxicity), PBMCs (a mixture of T cells and NK cells), and macrophages. The escape function of iPSC-DKO+MCL1 cells against different immune cells was assessed by RTCA. See Example 2.3 for the specific method.

### 5.1. Resistance of iPSC-DKO+MCL1 cells to NK cell killing

An NK cell killing assay was performed on an XCelligence platform (ACEA BioSciences). Various types of iPSCs (iPSC-WT cells, iPSC-DKO cells, and iPSC-DKO+MCL1 cells) were each resuspended in 100 µL of a cell-specific culture medium and plated onto a 96-well E-plate (ACEA BioSciences) coated with Matrigel (Sigma-Aldrich). After the cell index value reached 1, NK cells were added at an E:T ratio of 1:1. Data were normalized and analyzed using RTCA software (ACEA). The results are shown in FIG. 13. The iPSC-DKO+MCL1 cells could significantly escape NK cell killing, and their escaping effect was better than that of iPSC-WT.

### 5.2. Resistance of iPSC-DKO+MCL1 cells to ADCC killing

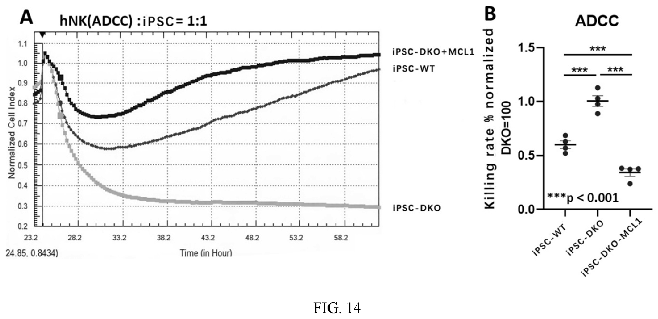

An NK cell ADCC killing assay was performed on an XCelligence platform (ACEA BioSciences). Various types of iPSCs were each resuspended in 100 µL of a cell-specific culture medium and plated onto a 96-well E-plate (ACEA BioSciences) coated with Matrigel (Sigma-Aldrich). After the cell index value reached 1, NK cells and an IgG Fc antibody were added at an E:T ratio of 1:1. Data were normalized and analyzed using RTCA software (ACEA). The results are shown in FIG. 14. The iPSC-DKO+MCL1 cells could significantly escape ADCC killing, and their escaping effect was better than that of iPSC-WT.

### 5.3. Resistance of DKO+MCL1 cells to PBMC killing

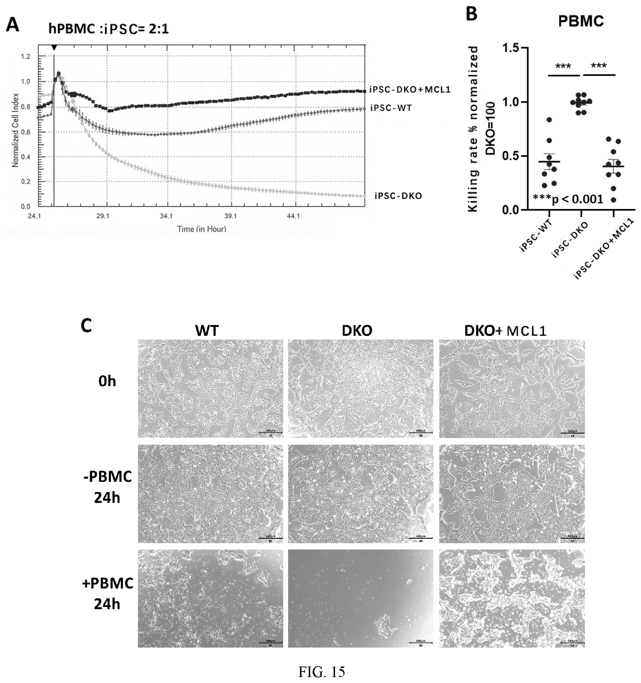

NK-activating factors were added to PBMCs in advance to improve the proportion of NK cells and T cell killing performance in PBMCs. RTCA experiments were conducted with the activated mixed lymphocytes (PBMCs) as effector cells to comprehensively evaluate the immune escape ability of iPSC-DKO+MCL1 cells (refer to PMID: 33309274 for the method). The specific procedure was as follows: A PBMC killing assay was performed on an XCelligence platform (ACEA BioSciences). Various types of iPSCs were each resuspended in 100 µL of a cell-specific culture medium and plated onto a 96-well E-plate (ACEA BioSciences) coated with Matrigel (Sigma-Aldrich). After the cell index value reached 1, PBMCs were added at an E:T ratio of 2:1. Data were normalized and analyzed using RTCA software (ACEA). The results are shown in A and B of FIG. 15. The iPSC-DKO+MCL1 cells could significantly escape PBMC killing, and their escaping effect was better than that of iPSC-WT.

In addition, WT, iPSC-DKO, and iPSC-DKO+MCL1 cells of iPSCs were plated onto 12-well plates. After 24 h, the culture medium was discarded, and PBMCs were added, and the cells were cultured. The results are shown in C of FIG. 15. Compared to the iPSC-WT and iPSC-DKO cells, fewer iPSC-DKO+MCL1 cells were killed by PBMCs, indicating that the ability of the iPSC-DKO+MCL1 cells to escape PBMC killing was significantly higher than that of the iPSC-WT and iPSC-DKO cells.

### 5.4. Resistance of iPSC-DKO+MCL1 cells to macrophage killing

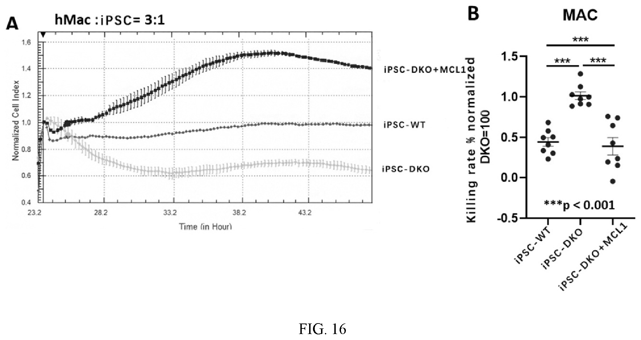

An MAC cell killing assay was performed on an XCelligence platform (ACEA BioSciences). Various types of iPSCs were each resuspended in 100 µL of a cell-specific culture medium and plated onto a 96-well E-plate (ACEA BioSciences) coated with Matrigel (Sigma-Aldrich). After the cell index value reached 1, MAC cells were added at an E:T ratio of 3:1. Data were normalized and analyzed using RTCA software (ACEA). The results are shown in FIG. 16. The iPSC-DKO+MCL1 cells could significantly escape macrophage killing, and their escaping effect was better than that of the iPSC-WT and iPSC-DKO cells.

### Example 6. Stemness of iPSC-DKO+MCL1 Cell Line

In this example, whether the stemness of iPSC-derived DKO+MCL1 pluripotent stem cells changed was further determined.

### 6.1. Expression of pluripotency genes in DKO cells

The protein levels of pluripotency genes in iPSC-DKO+MCL1 cells were determined by immunofluorescence, and the expression of the proteins corresponding to the pluripotency genes on the surface of iPSC-DKO+MCL1 cells was assessed by flow cytometry. The specific methods were as follows: Immunofluorescence assay: iPSC-DKO+MCL1 cells were plated onto a 12-well plate. After the cells grew to a density of 60-80%, the culture medium was discarded using a pipette, and 4% paraformaldehyde was added for fixation. After cell membrane permeabilization, the cells were incubated with primary antibodies of OCT4 and NANOG, as well as other pluripotency gene proteins, at 4 °C overnight, washed to remove the primary antibodies, and then incubated with fluorescently labeled secondary antibodies at room temperature. Subsequently, photos were taken using a fluorescence microscope (Nikon Ts2R-FL).

### Flow cytometry:

Cells were collected and then incubated with antibodies in an EP tube in a dark place at 4 °C for 30 min. Then, an appropriate fluorescence acquisition channel was selected based on antibody information, and fluorescence was measured on the instrument. All antibodies were from BD Biosciences.

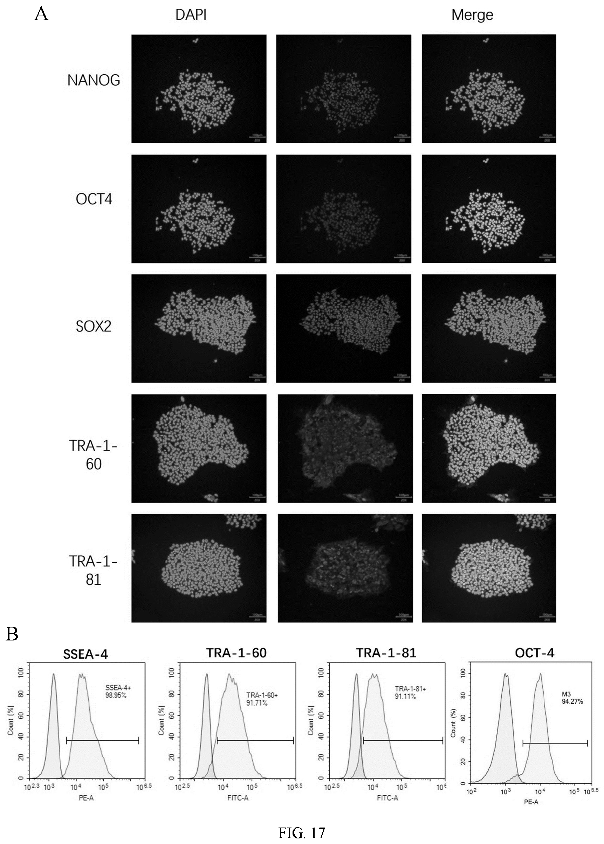

A of FIG. 17 shows the results of the determination of the protein levels of the NANOG, OCT4, SOX2, TRA-1-60, and TRA-1-81 pluripotency genes in iPSC-DKO+MCL1 cells by immunofluorescence, and B of FIG. 17 shows the results of the assessment of the expression of the SSEA-4, TRA-1-60, TRA-1-81, and OCT-4 pluripotency genes on the surface of iPSC-DKO+MCL1 cells by flow cytometry.

### 6.2. Ability of iPSC-DKO+MCL1 cell line to differentiate into three germ layers

The ability of iPSC-DKO+MCL1 cells to differentiate into three germ layers was assessed. To generate mesodermal, endodermal, and ectodermal cells, dissociated iPSC-DKO+MCL1 single cells were resuspended in three germ layer culture media supplemented with Y27632, and an appropriate number of cells were attached to Matrigel-coated well plates with coverslips. After 24 h, the culture media were replaced with preheated differentiation culture media, and the culture media were refreshed daily until the seventh day to obtain mesodermal, endodermal, and ectodermal cells. The expression of marker proteins of the three germ layers was assessed by immunofluorescence to assess the ability of iPSC-DKO+MCL1 cells to differentiate into the three germ layers.

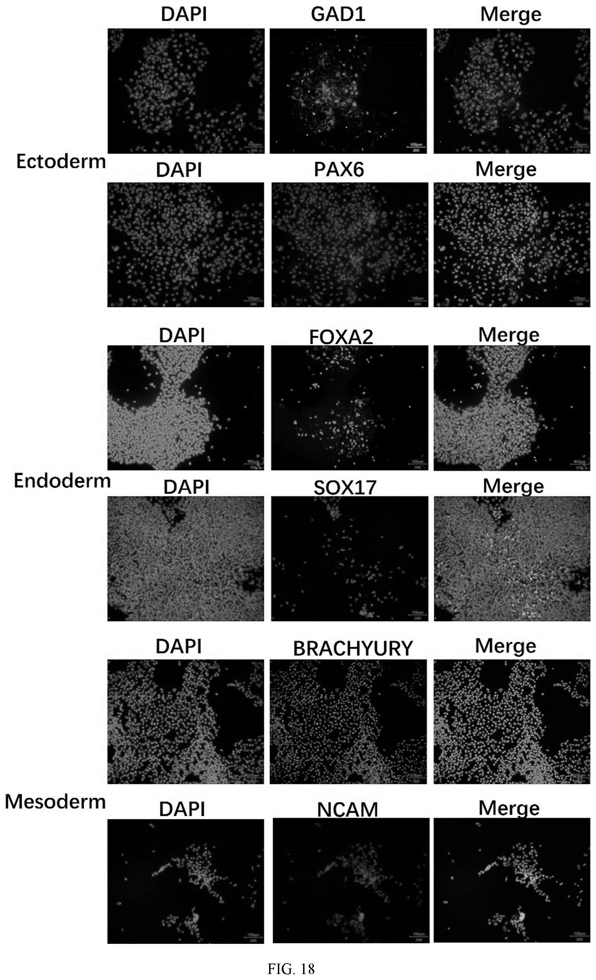

The immunofluorescence assay results are shown in FIG. 18. The iPSC-DKO+MCL1 cells expressed, at a protein level, PAX6 and GAD1, which are marker proteins of the ectoderm, Brachyury and NCAM, which are marker proteins of the mesoderm, and SOX17 and FOXA2, which are marker proteins of the endoderm.

### 6.3. Differentiation ability of DKO cells

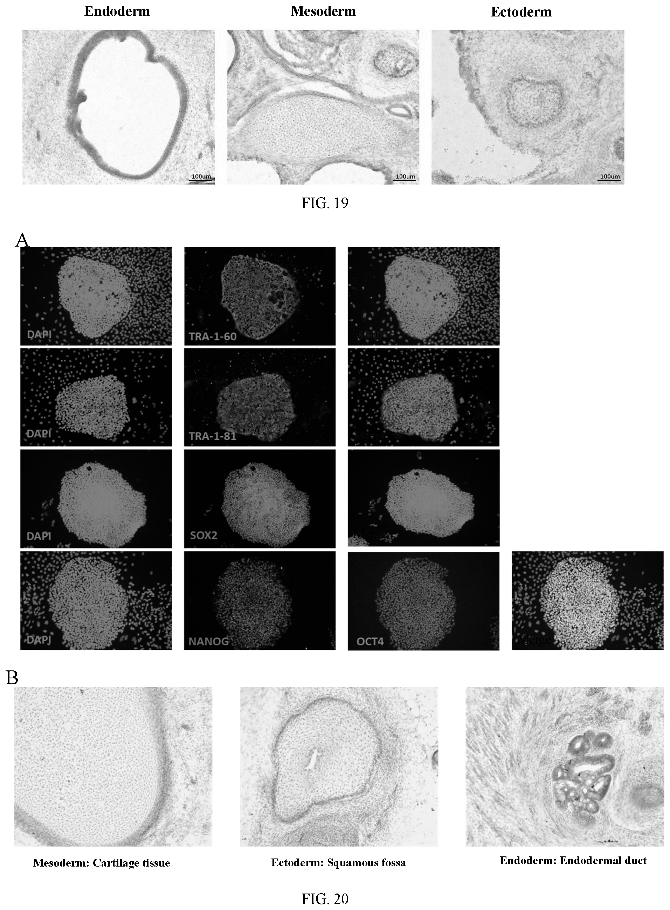

In this example, the differentiation ability of iPSC-DKO+MCL1 cells was assessed. The specific method was as follows: Immunodeficient mice (SCID Beige, Charles River) were each subcutaneously injected with 100 µL of a suspension containing 5 × 10⁵ iPSC-DKO+MCL1 cells. After the teratoma volume was greater than 1.5 cm³, the teratomas were collected and subjected to paraffin sectioning and hematoxylin and eosin staining. The staining results in FIG. 19 show that the iPSC-DKO+MCL1 cells could form teratomas *in vivo* and differentiate into cells of three germ layers (an endoderm, a mesoderm, and an ectoderm), indicating that the iPSC-DKO+MCL1 cells have the ability to normally differentiate into the three germ layers.

### Example 7. Construction and Validation of Universal NHP Cells

This example describes a study on the *in vivo* and *in vitro* immunogenicity of universal monkey stem cells (iPSCs) in a non-human primate (NHP) model.

### 1. Construction of universal NHP cells

### a) Construction of NHP iPSC-DKO cells

In this study, monkey iPSC-DKO cells (monkey iPSCs, prepared using a CTS^{™} CytoTune^{™}-iPS 2.1 Sendai virus reprogramming kit (Cat No.: A34546)) were produced according to the method described in Example 1. The expression of pluripotency genes in iPSCs and the formation of teratomas were assessed according to the methods described in Example 2. The results are shown in FIG. 20.

NHP-B2M-gRNA1 and NHP-B2M-gRNA2 were used to perform direct two-end knockout of the exons of B2M, and a genome sequence knockout validation was then performed using a pair of PCR primers: NHP-B2M-F/R. NHP-CIITA-gRNA1 and NHP-CIITA-gRNA2 were used to perform direct two-end knockout of the exons of CIITA, and a PCR validation of the genome knockout was performed using a pair of PCR primers: NHP-CIITA-F/R. Two NHP iPSC-DKO cell strains were selected for subsequent validations.
NHP-B2M-gRNA1 (SEQ ID NO: 7): CGTGAGTAAACCTGAATCTT
NHP-B2M-gRNA2 (SEQ ID NO: 8): AGTCACATGGTTCACACGGC
NHP-CIITA-gRNA1 (SEQ ID NO: 28): CATCGCTGTTGAGAAGCTCC
NHP-CIITA-gRNA2 (SEQ ID NO: 9): GATATTGGCATAAGCCTCCC

### B2M identification primers

NHP-B2M-F (SEQ ID NO: 29): CATTTGGCCAGAGTGGAAATG
NHP-B2M-R (SEQ ID NO: 30): TGGGACTCATTCAGGGTAGTA

### CIITA identification primers

NHP-CIITA-F (SEQ ID NO: 31): CTGTGAGGTGACTGAGCATATC
NHP-CIITA-R (SEQ ID NO: 32): GGCCAGCAATGAGCATACTA

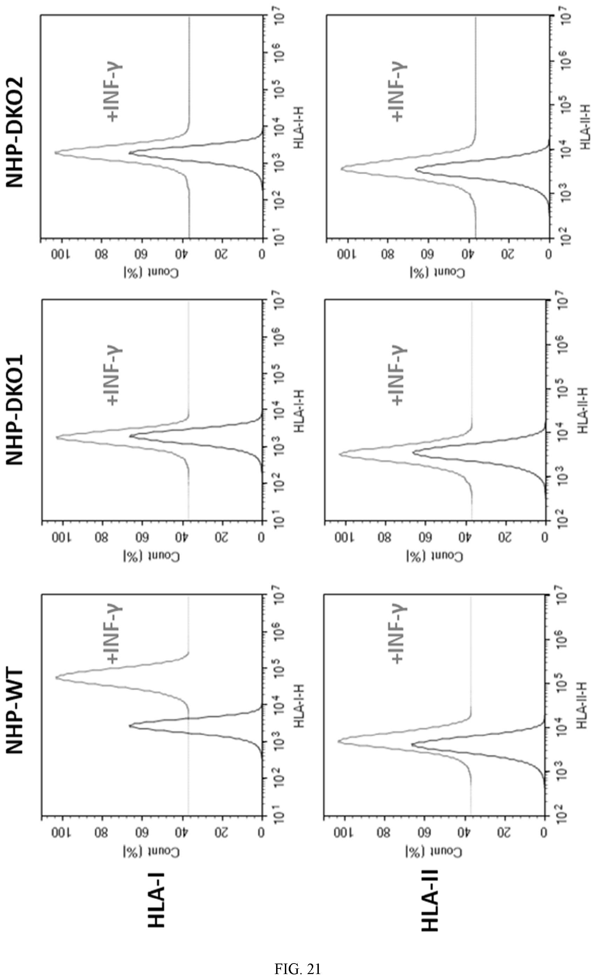

Stimulation of NHP iPSC-WT, NHP iPSC-DKO1, and NHP iPSC-DKO2 cells with IFN-gamma: The cells were plated onto well plates. The next day, at the time of refreshing the culture medium, a culture medium containing IFN-gamma was added to the cells. After 48 h, the cells were digested, and the expression of HLA-I/II was assessed by flow cytometry. The results are shown in FIG. 21. The B2M/CIITA double allele knockout NHP iPSC-DKO1 and NHP iPSC-DKO2 cells did not express HLA-I/II and could not up-regulate HLA-I/II in response to IFN-gamma stimulation.

### b) Functional validation of NHP iPSC-DKO cells escaping T cell killing

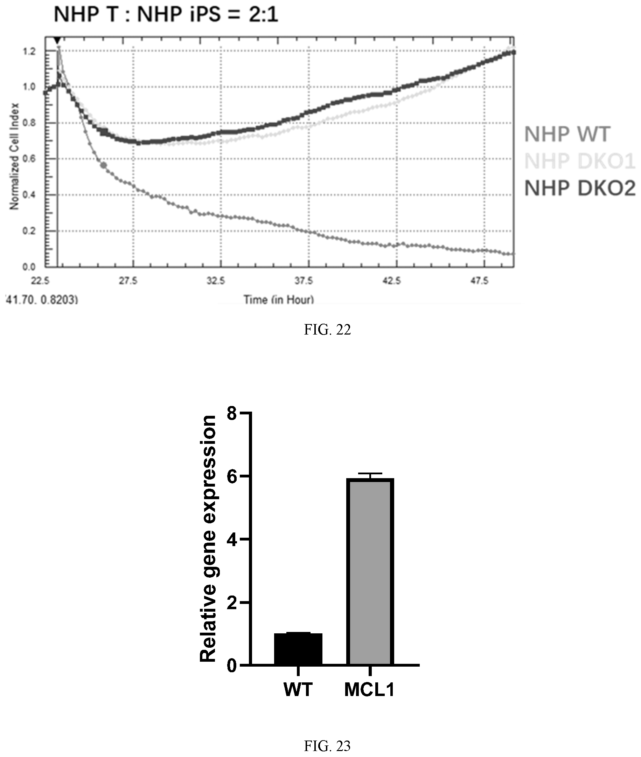

A T cell killing assay was performed on an XCelligence platform (ACEA BioSciences). NHP iPSC-WT and iPSC-DKO cells were resuspended in cell-specific culture media (100 µL), respectively, and plated onto 96-well E-plates (ACEA BioSciences) coated with Matrigel (Sigma-Aldrich). After the cell index value reached 1, T cells were added at an E:T ratio of 2:1. Data were normalized and analyzed using RTCA software (ACEA). The results are shown in FIG. 22. The NHP DKO cells could significantly escape T cell killing, while the NHP iPSC-WT cells were killed.

### 2. In vitro detection of universal NHP cells escaping immune system killing

NHP iPSC-DKO+MCL1 cells (monkey MCL1 gene sequence: XM_045364359.1) were prepared according to the method described above, and overexpression was confirmed by RT-qPCR The results are shown in FIG. 23.
NHP-MCL1-F (SEQ ID NO: 33): GCTGCATCGAACCATTAGCA
NHP-MCL1-R (SEQ ID NO: 34): ATGCCAAACCAGCTCCTACT

### a) Monkey NK cell killing

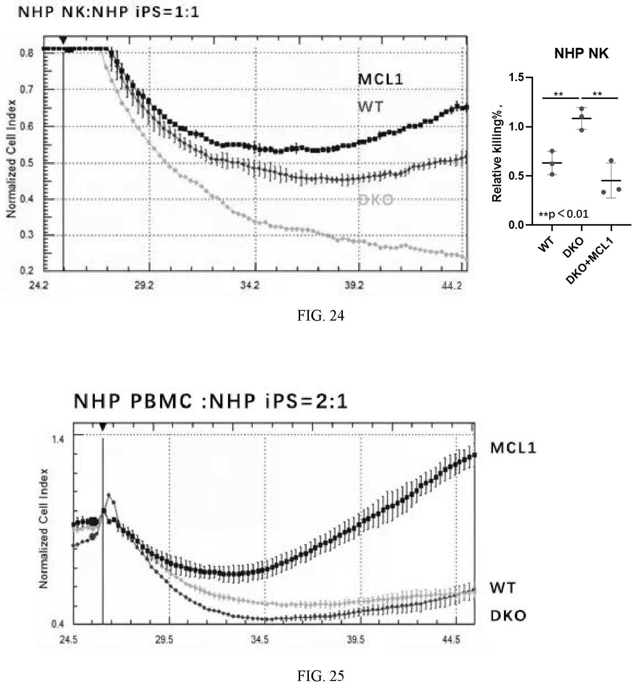

An NK cell killing assay was performed on an XCelligence platform (ACEA BioSciences). NHP iPSC-WT, NHP iPSC-DKO, and NHP iPSC-DKO+MCL1 cells were resuspended in cell-specific culture media (100 µL), respectively, and plated onto 96-well E-plates (ACEA BioSciences) coated with Matrigel (Sigma-Aldrich). After the cell index value reached 1, NK cells were added at an E:T ratio of 1:1. Data were normalized and analyzed using RTCA software (ACEA). The results are shown in FIG. 24. The NHP-WT and NHP iPSC-DKO+MCL1 cells could significantly escape monkey NK cell killing, and the ability to resist killing was more significant after MCL1 overexpression.

### b) Monkey PBMC killing

A PBMC killing assay was performed on an XCelligence platform (ACEA BioSciences). NHP iPSC-WT, NHP iPSC-DKO, and NHP iPSC-DKO+MCL1 cells were resuspended in cell-specific culture media (100 µL), respectively, and plated onto 96-well E-plates (ACEA BioSciences) coated with Matrigel (Sigma-Aldrich). After the cell index value reached 1, PBMCs were added at an E:T ratio of 2:1. Data were normalized and analyzed using RTCA software (ACEA). The results are shown in FIG. 25. The NHP iPSC-DKO+MCL1 cells could significantly escape PBMC killing, while NHP-WT and NHP-DKO cells were killed.

### SEQUENCE LISTING

| SEQ ID NO: | Description | Sequence |
|---|---|---|
| 1 | Amino acid sequence of B2M | MSRSVALAVLALLSLSGLEAIQRTPKIQVYSRHPAENGKSNFLNCYVSGF HPSDIEVDLLKNGERIEKVEHSDLSFSKDWSFYLLYYTEFTPTEKDEYAC RVNHVTLSQPKIVKWDRDM |
| 2 | Amino acid sequence of CIITA | MRCLAPRPAGSYLSEPQGSSQCATMELGPLEGGYLELLNSDADPLCLYH FYDQMDLAGEEEIELYSEPDTDTINCDQFSRLLCDMEGDEETREAYANIA ELDQYVFQDSQLEGLSKDIFKHIGPDEVIGESMEMPAEVGQKSQKRPFPE ELPADLKHWKPAEPPTVVTGSLLVGPVSDCSTLPCLPLPALFNQEPASGQ MRLEKTDQIPMPFSSSSLSCLNLPEGPIQFVPTISTLPHGLWQISEAGTGVS SIFIYHGEVPQASQVPPPSGFTVHGLPTSPDRPGSTSPFAPSATDLPSMPEP ALTSRANMTEHKTSPTQCPAAGEVSNKLPKWPEPVEQFYRSLQDTYGAE PAGPDGILVEVDLVQARLERSSSKSLERELATPDWAERQLAQGGLAEVLL AAKEHRRPRETRVIAVLGKAGQGKSYWAGAVSRAWACGRLPQYDFVFS VPCHCLNRPGDAYGLQDLLFSLGPQPLVAADEVFSHILKRPDRVLLILDG FEELEAQDGFLHSTCGPAPAEPCSLRGLLAGLFQKKLLRGCTLLLTARPR GRLVQSLSKADALFELSGFSMEQAQAYVMRYFESSGMTEHQDRALTLL RDRPLLLSHSHSPTLCRAVCQLSEALLELGEDAKLPSTLTGLYVGLLGRA ALDSPPGALAELAKLAWELGRRHQSTLQEDQFPSADVRTWAMAKGLVQ HPPRAAESELAFPSFLLQCFLGALWLALSGEIKDKELPQYLALTPRKKRP YDNWLEGVPRFLAGLIFQPPARCLGALLGPSAAASVDRKQKVLARYLK RLQPGTLRARQLLELLHCAHEAEEAGIWQHVVQELPGRLSFLGTRLTPP DAHVLGKALEAAGQDFSLDLRSTGICPSGLGSLVGLSCVTRFRAALSDT VALWESLQQHGETKLLQAAEEKFTIEPFKAKSLKDVEDLGKLVQTQRTR SSSEDTAGELPAVRDLKKLEFALGPVSGPQAFPKLVRILTAFSSLQHLDLD ALSENKIGDEGVSQLSATFPQLKSLETLNLSQNNITDLGAYKLAEALPSL AASLLRLSLYNNCICDVGAESLARVLPDMVSLRVMDVQYNKFTAAGAQ QLAASLRRCPHVETLAMWTPTIPFSVQEHLQQQDSRISLR |
| 3 | Amino acid sequence of MCL1 | MFGLKRNAVIGLNLYCGGAGLGAGSGGATRPGGRLLATEKEASARREIG GGEAGAVIGGSAGASPPSTLTPDSRRVARPPPIGAEVPDVTATPARLLFFAP TRRAAPLEEMEAPAADAIMSPEEELDGYEPEPLGKRPAVLPLLELVGESG NNTSTDGSLPSTPPPAEEEEDELYRQSLEIISRYLREQATGAKDTKPMGRS GATSRKALETLRRVGDGVQRNHETAFQGMLRKLDIKNEDDVKSLSRVM IHVFSDGVTNWGRIVTLISFGAFVAKHLKTINQESCIEPLAESITDVLVRTK RDWLVKQRGWDGFVEFFHVEDLEGGIRNVLLAFAGVAGVGAGLAYLIR |
| 4 | Nucleic acid sequence of MCL1 | ATGTTTGGCCTCAAAAGAAACGCGGTAATCGGACTCAACCTCTACTGT GGGGGGGCCGGCTTGGGGGCCGGCAGCGGCGCCACCCGCCCGG GAGGGCGACTTTTGGCTACGGAGAAGGAGGCCTCGGCCCGGCGAGA GATAGGGGGAGGGGAGGCCGGCGCGGTGATTGGCGGAAGCGCCGGC GCAAGCCCCCCGTCCACCCTCACGCCAGACTCCCGGAGGGTCGCGCG GCCGCCGCCCATTGGCGCCGAGGTCCCCGACGTCACCGCGACCCCCG CGAGGCTGCTTTTCTTCGCGCCCACCCGCCGCGCGGCGCCGCTTGAG GAGATGGAAGCCCCGGCCGCTGACGCCATCATGTCGCCCGAAGAGGA GCTGGACGGGTACGAGCCGGAGCCTCTCGGGAAGCGGCCGGCTGTC CTGCCGCTGCTGGAGTTGGTCGGGGAATCTGGTAATAACACCAGTAC GGACGGGTCACTACCCTCGACGCCGCCGCCAGCAGAGGAGGAGGAG GACGAGTTGTACCGGCAGTCGCTGGAGATTATCTCTCGGTACCTTCGG GAGCAGGCCACCGGCGCCAAGGACACAAAGCCAATGGGCAGGTCTG GGGCCACCAGCAGGAAGGCGCTGGAGACCTTACGACGGGTTGGGGA TGGCGTGCAGCGCAACCACGAGACGGCCTTCCAAGGCATGCTTCGGA AACTGGACATCAAAAACGAAGACGATGTGAAATCGTTGTCTCGAGTG ATGATCCATGTTTTCAGCGACGGCGTAACAAACTGGGGCAGGATTGTG ACTCTCATTTCTTTTGGTGCCTTTGTGGCTAAACACTTGAAGACCATA AACCAAGAAAGCTGCATCGAACCATTAGCAGAAAGTATCACAGACGT TCTCGTAAGGACAAAACGGGACTGGCTAGTTAAACAAAGAGGCTGG GATGGGTTTGTGGAGTTCTTCCATGTAGAGGACCTAGAAGGTGGCATC AGGAATGTGCTGCTGGCTTTTGCAGGTGTTGCTGGAGTAGGAGCTGG TTTGGCATATCTAATAAGATAG |
| 5 | Amino acid sequence of CD47 | MWPLVAALLLGSACCGSAQLLFNKTKSVEFTFCNDTVVIPCFVTNMEAQ NTTEVYVKWKFKGRDIYTFDGALNKSTVPTDFSSAKIEVSQLLKGDASL KMDKSDAVSHTGNYTCEVTELTREGETIIELKYRVVSWFSPNENILIVIFPI F AILLFWGQFGIKTLKYRSGGMDEKTIALLVAGLVITVIVIVGAILFVPGEY SLKNATGLGLIVTSTGILILLHYYVFSTAIGLTSFVIAILVIQVIAYILAVVGL SLCIAACIPMHGPLLISGLSILALAQLLGLVYMKFVASNQKTIQPPRNN |
| 6 | Nucleic acid sequences encoding CD47 protein | ATGTGGCCCCTGGTAGCGGCGCTGTTGCTGGGCTCGGCGTGCTGCGG ATCAGCTCAGCTACTATTTAATAAAACAAAATCTGTAGAATTCACGTTT TGTAATGACACTGTCGTCATTCCATGCTTTGTTACTAATATGGAGGCAC AAAACACTACTGAAGTATACGTAAAGTGGAAATTTAAAGGAAGAGAT ATTTACACCTTTGATGGAGCTCTAAACAAGTCCACTGTCCCCACTGAC TTTAGTAGTGCAAAAATTGAAGTCTCACAATTACTAAAAGGAGATGCC TCTTTGAAGATGGATAAGAGTGATGCTGTCTCACACACAGGAAACTA CACTTGTGAAGTAACAGAATTAACCAGAGAAGGTGAAACGATCATCG AGCTAAAATATCGTGTTGTTTCATGGTTTTCTCCAAATGAAAATATTCT TATTGTTATTTTCCCAATTTTTGCTATACTCCTGTTCTGGGGACAGTTTG GTATTAAAACACTTAAATATAGATCCGGTGGTATGGATGAGAAAACAA TTGCTTTACTTGTTGCTGGACTAGTGATCACTGTCATTGTCATTGTTGG AGCCATTCTTTTCGTCCCAGGTGAATATTCATTAAAGAATGCTACTGGC CTTGGTTTAATTGTGACTTCTACAGGGATATTAATATTACTTCACTACTA TGTGTTTAGTACAGCGATTGGATTAACCTCCTTCGTCATTGCCATATTG GTTATTCAGGTGATAGCCTATATCCTCGCTGTGGTTGGACTGAGTCTCT GTATTGCGGCGTGTATACCAATGCATGGCCCTCTTCTGATTTCAGGTTT GAGTATCTTAGCTCTAGCACAATTACTTGGACTAGTTTATATGAAATTT GTGGCTTCCAATCAGAAGACTATACAACCTCCTAGGAATAACTGA |

Although the present disclosure has been disclosed above by preferred examples, the examples are not intended to limit the present disclosure. Those skilled in the art can make various changes and modifications without departing from the spirit and scope of the present disclosure. Therefore, the scope of the present disclosure should be defined by the claims.

## Claims

1. A hypoimmunogenic pluripotent stem cell, comprising:
reduced MHC-I function compared to a parental pluripotent stem cell;
reduced MHC-II function compared to the parental pluripotent stem cell; and
reduced sensitivity to T cell and NK cell killing compared to the parental pluripotent stem cell, wherein the reduced sensitivity to T cell and NK cell killing results from increased expression of an MCL1 protein.

2. The hypoimmunogenic pluripotent stem cell according to claim 1, wherein the cell further comprises a modification to increase expression of one or more of the following polypeptides: DUX4, CD27, CD35, CD200, PD-L1, CD47, CD24, CD26, CCL21, Mfge8, and SerpinB9.

3. The hypoimmunogenic pluripotent stem cell according to claim 1 or 2, wherein the MHC-I function is reduced by reducing activity of an MHC class I protein or an MHC-I transcription factor.

4. The hypoimmunogenic pluripotent stem cell according to claim 3, wherein the MHC class I protein comprises an HLA-A protein, an HLA-B protein, or an HLA-C protein.

5. The hypoimmunogenic pluripotent stem cell according to claim 3, wherein the MHC-I transcription factor comprises a B2M protein, a TAP1 protein, a TAP2 protein, a TAP-related glycoprotein, or an NLRC5 protein.

6. The hypoimmunogenic pluripotent stem cell according to claim 3, wherein the MHC-I function is reduced by reducing activity of a B2M protein.

7. The hypoimmunogenic pluripotent stem cell according to claim 6, wherein the B2M protein is a human B2M protein comprising the amino acid sequence set forth in SEQ ID NO: 1 or an amino acid sequence having at least 90% identity to the amino acid sequence set forth in SEQ ID NO: 1;
or the B2M protein is a monkey B2M protein comprising the amino acid sequence with accession No. XP_045251305.2 or an amino acid sequence having at least 90% identity to the amino acid sequence with accession No. XP_045251305.2.

8. The hypoimmunogenic pluripotent stem cell according to any one of claims 1-7, wherein the MHC-II function is reduced by reducing activity of an MHC class II protein or an MHC-II transcription factor.

9. The hypoimmunogenic pluripotent stem cell according to claim 8, wherein the MHC class II protein comprises an HLA-DR protein, an HLA-DQ protein, or an HLA-DP protein.

10. The hypoimmunogenic pluripotent stem cell according to claim 8, wherein the MHC-II transcription factor comprises a CIITA protein, an RFXANK protein, an RFX5 protein, or an RFXAP protein.

11. The hypoimmunogenic pluripotent stem cell according to claim 8, wherein the MHC-II function is reduced by reducing activity of a CIITA protein.

12. The hypoimmunogenic pluripotent stem cell according to claim 11, wherein the CIITA protein is a human CIITA protein comprising the amino acid sequence set forth in SEQ ID NO: 2 or an amino acid sequence having at least 90% identity to the amino acid sequence set forth in SEQ ID NO: 2;
or the CIITA protein is a monkey CIITA protein comprising the amino acid sequence with accession No. XP_005591301.3 or an amino acid sequence having at least 90% identity to the amino acid sequence with accession No. XP_005591301.3.

13. The hypoimmunogenic pluripotent stem cell according to any one of claims 1-12, wherein the MCL1 protein is a human MCL1 protein whose protein sequence comprises the amino acid sequence set forth in SEQ ID NO: 3 or an amino acid sequence having at least 90% identity to the amino acid sequence set forth in SEQ ID NO: 3;
or the MCL1 protein is a monkey MCL1 protein comprising the amino acid sequence with accession No. XP_045220294.2 or an amino acid sequence having at least 90% identity to the amino acid sequence with accession No. XP_045220294.2.

14. The hypoimmunogenic pluripotent stem cell according to claim 1, comprising:
one or more alterations that reduce activity of an endogenous B2M protein;
one or more alterations that reduce activity of an endogenous CIITA protein; and
one or more alterations that result in the increased expression of the MCL1 protein in the hypoimmunogenic pluripotent stem cell.

15. The hypoimmunogenic pluripotent stem cell according to claim 14, comprising:
one or more alterations that inactivate two alleles of an endogenous B2M gene;
one or more alterations that inactivate two alleles of an endogenous CIITA gene; and
one or more alterations that result in increased expression of an MCL1 gene in the hypoimmunogenic pluripotent stem cell.

16. A method for producing the hypoimmunogenic pluripotent stem cell according to any one of claims 1-15, comprising:
reducing the MHC-I function in the pluripotent stem cell;
reducing the MHC-II function in the pluripotent stem cell; and
increasing expression of a protein that reduces sensitivity of the pluripotent stem cell to NK cell, T cell, macrophage, and/or ADCC killing, wherein the protein is the MCL1 protein.

17. The method according to claim 16, comprising:
reducing activity of a B2M protein in the pluripotent stem cell;
reducing activity of a CIITA protein in the pluripotent stem cell; and
increasing the expression of the MCL1 protein in the pluripotent stem cell.

18. The method according to claim 17, comprising:
eliminating activity of two alleles of a B2M gene in the pluripotent stem cell;
eliminating activity of two alleles of a CIITA gene in the pluripotent stem cell; and
increasing expression of an MCL1 gene in the pluripotent stem cell.

19. The method according to any one of claims 16-18, wherein the hypoimmunogenic pluripotent stem cell further expresses a gene sequence of one selected from DUX4, CD27, CD35, CD200, PD-L1, CD47, CD24, CD26, CCL21, Mfge8, and SerpinB9.

20. The method according to any one of claims 16-19, wherein activity of a B2M protein in the pluripotent stem cell is reduced and/or activity of a CIITA protein in the pluripotent stem cell is reduced by a technology selected from the following:
introduction of a gene expression-modifying molecule, CRISPR technology, transcription activator-like effector nuclease technology, zinc finger nuclease technology, and homologous recombination technology; preferably, the gene expression-modifying molecule comprises an siRNA, an shRNA, a microRNA, an antisense RNA, an antisense oligonucleotide ASO, or an anti-miRNA oligonucleotide AMO.

21. The method according to claim 20, wherein the activity of the B2M protein in the pluripotent stem cell is reduced by CRISPR/Cas9 gene editing technology; and/or the activity of the CIITA protein in the pluripotent stem cell is reduced by CRISPR/Cas9 gene editing technology.

22. The method according to any one of claims 16-20, wherein the expression of the MCL1 protein is increased by modifying an endogenous locus.

23. The method according to any one of claims 16-20, wherein the expression of the MCL1 protein is increased by expression of a transgene; preferably, the expression of the MCL1 protein is increased by synthesizing a nucleic acid sequence encoding the MCL1 protein, constructing the nucleic acid sequence into a lentiviral vector, and then introducing at least one copy of an MCL1 gene under the control of a promoter into the pluripotent stem cell by the lentiviral vector.

24. The method according to claim 23, wherein the nucleic acid sequence encoding the MCL1 protein comprises the nucleic acid sequence set forth in SEQ ID NO: 4 or a nucleic acid sequence having at least 80% identity to the nucleic acid sequence set forth in SEQ ID NO: 4;
or the nucleic acid sequence encoding the MCL1 protein comprises the nucleic acid sequence with accession No. XM_045364359.1 or a nucleic acid sequence having at least 90% identity to the nucleic acid sequence with accession No. XM_045364359.1.

25. A composition, comprising the hypoimmunogenic pluripotent stem cell according to any one of claims 1-15 or a hypoimmunogenic pluripotent stem cell prepared by the method according to any one of claims 16-24, wherein preferably, the composition further comprises a pharmaceutically acceptable carrier.

26. Use of the hypoimmunogenic pluripotent stem cell according to any one of claims 1-15, a hypoimmunogenic pluripotent stem cell prepared by the method according to any one of claims 16-24, or the composition according to claim 25 in the preparation of a medicament for preventing or treating a disease requiring cell transplantation, preferably, the disease comprises acute leukemia, chronic leukemia, lymphoma, myelodysplastic syndrome, multiple myeloma, small cell lung cancer, breast cancer, testicular cancer, neuroblastoma, ovarian cancer, melanoma, aplastic anemia, primary immunodeficiency, systemic lupus erythematosus, rheumatoid arthritis, ankylosing spondylitis, type I diabetes, Parkinson's disease, Alzheimer's disease, spinal cord injury, retinal degenerative disease, stroke, Huntington's disease, amyotrophic lateral sclerosis, atherosclerosis, hypertension, rheumatic heart disease, cardiomyopathy, arrhythmia, congenital heart disease, valvular heart disease, carditis, myocardial infarction, heart failure, aortic aneurysm, peripheral artery disease, type II diabetes, scurvy, hypoglycemia, hyperlipidemia, or osteoporosis.

27. Use of the hypoimmunogenic pluripotent stem cell according to any one of claims 1-15, the hypoimmunogenic pluripotent stem cell prepared by the method according to any one of claims 16-24, or the composition according to claim 25, in the prevention or treatment of diseases requiring cell transplantation; preferably, the diseases comprises acute leukemia, chronic leukemia, lymphoma, myelodysplastic syndromes, multiple myeloma, small cell lung cancer, breast cancer, testicular cancer, neuroblastoma, ovarian cancer, melanoma, aplastic anemia, congenital immunodeficiency, systemic lupus erythematosus, rheumatoid arthritis, ankylosing spondylitis, type I diabetes, Parkinson's disease, Alzheimer's disease, spinal cord injury, retinal degenerative diseases, stroke, Huntington's disease, amyotrophic lateral sclerosis, atherosclerosis, hypertension, rheumatic heart disease, cardiomyopathy, arrhythmia, congenital heart disease, valvular heart disease, carditis, myocardial infarction, heart failure, aortic aneurysm, peripheral arterial disease, type II diabetes, septicemia, hypoglycemia, hyperlipidemia, or osteoporosis.

28. A hypoimmunogenic pluripotent stem cell according to any one of claims 1-15, a hypoimmunogenic pluripotent stem cell prepared by the method according to any one of claims 16-24, or a composition according to claim 25, for use in the prevention or treatment of diseases requiring cell transplantation; preferably, the diseases comprises acute leukemia, chronic leukemia, lymphoma, myelodysplastic syndromes, multiple myeloma, small cell lung cancer, breast cancer, testicular cancer, neuroblastoma, ovarian cancer, melanoma, aplastic anemia, congenital immunodeficiency, systemic lupus erythematosus, rheumatoid arthritis, ankylosing spondylitis, type I diabetes, Parkinson's disease, Alzheimer's disease, spinal cord injury, retinal degenerative diseases, stroke, Huntington's disease, amyotrophic lateral sclerosis, atherosclerosis, hypertension, rheumatic heart disease, cardiomyopathy, arrhythmia, congenital heart disease, valvular heart disease, carditis, myocardial infarction, heart failure, aortic aneurysm, peripheral arterial disease, type II diabetes, septicemia, hypoglycemia, hyperlipidemia, or osteoporosis.
